# EUROPÄISCHE PATENTSCHRIFT

(11) **EP 3 212 133 B1**
(45) Veröffentlichungstag und Bekanntmachung des Hinweises auf die Patenterteilung: **25.03.2020**
(21) Anmeldenummer: 15808452.5
(22) Anmeldetag: 29.10.2015
(51) Int. Cl.: A61F 5/00

(54) **BYPASS-VORRICHTUNG ZUR TRANS-PYLORISCHEN DURCHLEITUNG VON MAGENINHALT IN BZW. DURCH DEN ZWÖLFFINGERDARM SOWIE APPLIKATOR ZUR POSITIONIERUNG DESSELBEN**
BYPASS DEVICE FOR PASSING STOMACH CONTENT THROUGH THE PYLORUS INTO OR THROUGH THE DUODENUM AND POSITION DEVICE
OUTIL DE PONTAGE POUR PASSER UN CONTENU DE L'ESTOMAC A TRAVERS LE PYLORE DANS OU A TRAVERS LE DUODÉNUM ET OUTIL DE POSITIONNEMENT

(30) Priorität: 29.10.2014 DE 102014015919
(43) Veröffentlichungstag der Anmeldung: 06.09.2017
(73) Patentinhaber: Trans-Duodenal Concepts UG, 68753 Waghäusel (DE)
(72) Erfinder: GÖBEL, Fred, 69259 Wilhelmsfeld (DE)
(74) Vertreter: Küchler, Stefan
(86) Internationale Anmeldenummer: PCT/IB2015/002005
(87) Internationale Veröffentlichungsnummer: WO 2016/067087

(56) Entgegenhaltungen:
- WO-A1-2008/106041
- WO-A1-2011/099940
- WO-A1-2013/129088
- WO-A1-2014/089129
- US-A1- 2005 273 060
- US-A1- 2011 082 535

## Beschreibung

Die Erfindung richtet sich auf eine trans-pylorisch platzierbare Bypass-Vorrichtung zur Aufnahme von Nahrungsbrei aus dem Magen oder Gaster und zur bypass-artigen Fortleitung des Nahrungsbreis durch den Magenpförtner oder Pylorus in oder durch den Zwölffingerdarm oder das Duodenum eines Patienten, umfassend ein schlauchförmiges, vorzugsweise radial kollabierbares und sich selbst aufrichtendes, den Pylorus durchsetzendes, trans-pylorisches Durchleitungselement mit einem zentralen Durchleitungslumen für den Nahrungsbrei, sowie Fixierungselemente zur Verankerung des trans-pylorischen Durchleitungselements an dem Pylorus, bestehend aus einem magenseitig oder proximal des Pylorus angeordneten, ringförmigen, gastrischen Ankerelement zur Verankerung des trans-pylorischen Durchteitungseiements proximal des Pylorus, mit einem gastrischen Ballonsegment, welches einen mit einem Medium befüllbaren Hohlraum von ringförmiger Struktur bereichsweise umgrenzt, sowie aus einem darmseitig oder distal des Pylorus im Zwölffingerdarm liegenden, ringförmigen, duodenalen Ankerelement zur Verankerung des trans-pylorischen Durchleitungselements distal des Pylorus, mit einem duodenalen Ballonsegment, welches einen mit einem Medium befüllbaren Hohlraum von ringförmiger Struktur bereichsweise umgrenzt; sowie auf einen Applikator zur Platzierung einer solchen Bypass-Vorrichtung in einem Patienten. Dokument US 2005/0273060 offenbart ein Durchleitungselement mit einem Ballonsegment.

In Deutschland sind ca. 90 % aller Diabetiker, somit 4,5 Mio. Menschen, vom Typ-2-Diabetes betroffen, welcher meist durch Übergewicht (Adipositas) verursacht oder begleitet wird. Dies schränkt die Lebensqualität der Betroffenen ein und löst zahlreiche Folgeerkrankungen aus. Industrie-, Schwellen- und Entwicklungsländer sind hierbei in ähnlicher Weise betroffen. Entsprechend geht man weltweit in den kommenden Jahren von einer signifikanten Zunahme ernährungsbedingter Diabetes-Erkrankungen aus.

Bisherige Therapien zielen überwiegend auf eine medikamentöse Regulation der Stoffwechsellage, eine kausale Therapie im eigentlichen Sinne gibt es nicht. Neben der medikamentösen Stoffwechselregulation haben sich bei adipösen, an Diabetes leidenden Patienten verschiedene Verfahren der Adipositaschirurgie etabliert. Bei diesen wird grundsätzlich zwischen restriktiven (Magenband, Magenverkleinerung, Magenballon) und malabsorptiven Verfahren (Bypass, Duodenal-Switch, biliopankreatische Diversion) unterschieden, wobei die größten und nachhaltigsten Erfolge durch eine Kombination beider Verfahrensprinzipien erzielt werden.

Neuere Erfahrungen in der Adipositaschirurgie haben gezeigt, dass insbesondere Operations-Verfahren, die einen Bypass zum Zwölffingerdarm (Duodenum) herstellen, über die Gewichtsreduktion hinaus einen direkten und sofortigen Effekt auf die diabetische Stoffwechsellage haben, so dass diese Verfahren mit zunehmender Relevanz bei der Therapie von Diabetikern angewendet werden.

Gegen eine massenhafte Anlage operativer Überbrückungen des Duodenums sprechen jedoch:
- die gravierenden und irreversiblen Veränderungen am Magen-Darm-Trakt, die durch diese Operationen geschaffen werden;
- die Tatsache, dass die Langzeiteffekte auf den Stoffwechsel, Knochenstabilität, Tumorentstehung unzureichend erforscht sind;
- dass es durch die Eingriffe zu seltenen, aber sehr schwerwiegenden und bisweilen tödlichen Komplikationen kommen kann;
- dass die Patienten eine auf lange Sicht sehr aufwändige Nachbetreuung mit Kontrollen von Stoffwechselparametern benötigen;
- dass erhebliche Behandlungskosten entstehen.

Wegen der mit operativen Verfahren verbundenen Komplikationen und Kosten hat sich die Endoskopie zum Ziel gesetzt, ein Implantat zur Therapie von Diabetes und Übergewicht zu entwickeln, das in Anlehnung an die duodenale Bypass-Chirurgie auf eine Durchleitung von Speisebrei durch den Zwölffingerdarm mittels einer im Duodenum platzierten Schlauchprothese basiert.

Die zur Verdauung bzw. zum Aufschluss von Nahrung erforderlichen Sekrete aus der Bauchspeicheldrüse und der Gallenblase fließen dem Nahrungsbrei normalerweise im Bereich des mittleren Duodenums zu. Es konnte im Rahmen klinischer Untersuchungen nachgewiesen werden, dass durch eine künstlich angelegte trans-duodenale Bypass-Prothese, welche den Nahrungsbrei im Magen aufnimmt und ohne Vermengung mit den verdauungsaktiven Sekreten des Duodenums auf direktem Wege in das beginnende Jejunum führt, der Aufschluss und damit die Resorption der Nahrungsbestandteile reduziert werden kann, was neben einer sich einstellenden Reduktion des Körpergewichtes unmittelbaren Einfluss auf den Blutzuckerspiegel und damit die Insulinausschüttung nimmt. Der genaue Mechanismus dieses Phänomens ist noch nicht erforscht, seine Wirksamkeit jedoch nachgewiesen.

Die konstruktive Gestaltung derartiger Bypass-Schlauchprothesen sieht in der Regel einen die Vorrichtung im Bereich des Magenausgangs bzw. Schließmuskels (Pylorus) verankernden Mechanismus vor, welcher die Nahrung möglichst vollständig im Magen oder im unmittelbaren Anschluss an den Magen aufnimmt. An dieses ankernde Teil schließt sich ein fortleitendes schlauchartiges Teil an, das den magenseitig aufgenommenen Nahrungsbrei durch das Duodenum in das beginnende Jejunum leitet. Der Nahrungsbrei wird dabei durch die propulsiven Bewegungen (Peristaltik) des Duodenums im Schlauch, in weitgehender Analogie zum natürlichen Transport, vorangetrieben.

Eine durch flexibel-endoskopische Implantation applizierbare Bypass-Technik würde sich durch die folgenden Vorzüge auszeichnen:
- minimale Invasivität;
- komfortable Reversibilität, weil es auf ebenfalls endoskopischem Weg wieder entfernt werden kann; sowie
- preisgünstige Anwendbarkeit.

Derzeit befinden sich bereits verschiedene endoskopisch platzierbare duodenale Bypass-Vorrichtungen mit dem Ziel der enteralen Resorptionsreduzierung in der Erprobung. Die Systeme werden, je nach baulicher Ausführung, entweder in einer sich dem Pylorus distal unmittelbar anschließenden Position im oberen Duodenum (Bulbus duodeni) verankert oder sie werden innerhalb des Schließmuskels bzw. über den Pylorus hinweg in den Magen reichend positioniert.

Unabhängig vom jeweiligen Bautyp, müssen die Verankerungsmechanismen einerseits gewährleisten, dass ein weitgehend flüssigkeitsdichter Abschluss der ankernden Kopfeinheit der Vorrichtung zur Duodenalwand bzw. zum Pylorus hin erreicht wird, um so effizient wie möglich zu vermeiden, dass Nahrungsbestandteile das Duodenum außerhalb der Schlauchprothese passieren und so deren Wirksamkeit einschränken. Andererseits müssen die Kräfte, die durch den ankernden Mechanismus auf die jeweilig anliegenden Organwandungen wirken, so weit reduziert werden, dass degenerative Veränderungen, die im Verlauf zu Blutungen oder Perforationen führen könnten, ausgeschlossen werden. Die Balance zwischen effizienter Ankerung und Dichtung sowie organverträglicher Platzierung ist nicht zuletzt wegen der besonderen Motilität der anatomischen Strukturen im Bereich des Übergangs vom Magen zum Duodenum eine Herausforderung. Der ankernde und dichtende Anteil des Bypass muss der kontraktilen Dynamik in möglichst kompatibler Weise folgen.

Mit dem Schwerpunkt der anti-diabetischen Therapie wird seit etwa zwei Jahren eine endoskopisch platzierte, trans-duodenale Schlauchprothese von ca. 60 cm Länge eingesetzt, die mit einem stent-artigen Metallkörbchen im oberen Duodenum innerhalb des sog. Bulbus duodeni verankert wird. Diese Verankerung des Körbchens erfolgt durch stachelartige Fortsätze, die sich in die Mucosa des Bulbus eingraben und beim Patienten zur Ursache heftiger intermittierender Schmerzen werden können. Die Technik erfordert eine permanente Einnahme von magensäurehemmenden Medikamenten und kann Komplikationen wie Blutungen und Perforationen der Duodenalwand verursachen. Insbesondere kann die Entfernung des Implantates wegen der seitlich abstehenden Metallstachel riskant sein.

Neben derartigen, stent-basierten Techniken zur Verankerung der den Speisebrei aufnehmenden Bypass-Vorrichtungen, sind unter anderem ballon- und ringbasierte, trans-pylorisch platzierte Ankersysteme in der Erprobung. Bei diesen wird der Pylorus, welcher den anatomischen Übergang vom Magen in das Duodenum markiert, zwischen zwei ballon- oder ringartige Strukturen in eine Art klemmende Dichtung genommen. Eine entsprechende, trans-pylorische Verankerung durch sich elastisch aufrichtende, O-Ring-artige Elemente, welche dem Pylorus magen- und duodenalseitig anliegen, wird in der US 5,820,584 beschrieben. Die beiden Ringelemente sind in das Durchleitungselement integriert und richten sich durch ihre elastische Eigenspannung jeweils proximal und distal des Schließmuskels auf. Die kanalartige Durchtrittsöffnung des Pylorus wird durch eine zwischen den Ringen entfaltete, ringförmig geschlossene Membran ausgekleidet. Am duodenalseitig platzierten Ring schließt sich zum Dünndarm hin eine schlauchartige Formation zur Fortleitung des Mageninhaltes durch das Duodenum an. Problematisch bei derartigen Techniken zur Verankerung ist die zur dislokationssicheren, trans-pylorischen Positionierung erforderliche, elastische Rückstellkraft der selbstaufrichtenden Ringkomponenten, die eine permanente kleinflächige Kraftwirkung auf die Gewebe beidseits des Ringmuskels ausübt. Diese anhaltende Kraftwirkung kann durch die erforderlichen langfristigen Anwendungszeiträume des trans-duodenalen Bypass ebenfalls zu druckbedingten Schädigungen bzw. Nekrosen führen. Zudem ist die endoskopische Platzierung solcher Ringelemente relativ schwierig.

Die US 2011/0004320 A1 beschreibt beispielhaft eine duodenale Bypass-Vorrichtung auf der Grundlage zweier reifenartiger, trans-pylorisch platzierter Befestigungselemente, welche durch strangartige Haltetrassen miteinander verbunden sind. Die Ringelemente weisen jeweils einen felgenartigen Innenreifen auf, dem ein elastisch aufdehnbares Ballonschlauchelement aufsitzt, welches sich bei Befüllung primär radial erweitert und so magen- und duodenalseitig als ankerndes Widerlager wirkt. Dabei gehen bei der Befüllung die ballonartig präformierten Widerlagerelemente, welche dem Pylorus beidseits anliegen, in den Zustand einer toroidalen, elastischen Aufdehnung über, mit einem etwa kreisförmigen Querschnitt, wobei der Pylorus selbst weitgehend unbelastet bleibt, weil die vollständig entfalteten Widerlagerelemente ihre Kräfte in erster Linie auf die dem Pylorus angrenzenden Anteile des Magens bzw. des Duodenums lenken. Die resultierende Belastung dieser Strukturen kann zu entsprechenden degenerativen Schädigungen, wie bei den zuvor beschriebenen, sich elastisch aufrichtenden Ringelementen führen.

Zur Erreichung einer optimal gewebe- und organverträglichen, dauerhaften Platzierung einer trans-pylorisch positionierten Bypass-Vorrichtung wäre eine Ankerwirkung wünschenswert, die die radiale Kraftentwicklung auf die dem Pylorus benachbarten Strukturen reduziert und ggf. auch, in axialer Richtung, auf den pylorischen Schließmuskel selbst lenkt.

Desweiteren sollte die trans-pylorische Ankerung in der Lage sein, sich funktionellen Änderungen des Schließmuskels so weit wie möglich selbstständig anzupassen. Im idealen Falle sollte die Motilität des Pylorus unbeeinträchtigt bleiben bzw. der Pylorus-Schluss und eine entsprechende Verformung der trans-pylorischen Anteile der Vorrichtung bei möglichst geringer Kontraktionskraft erfolgen können.

Ferner sollte es bei Bypässen des trans-pylorischen Typs möglich sein, die auf die pylorischen und dem Pylorus angrenzenden Strukturen lastende Kraft von außerhalb des Körpers einstellbar zu machen bzw. dem Individuum im Verlauf anzupassen.

Von Bedeutung für eine möglichst effizient gewichtsreduzierende Wirkung der Vorrichtung wären zudem Bypass-Vorrichtungen, welche die (malresorptive) Bypassfunktion mit anderen Wirkprinzipien, wie zum Beispiel eine (restriktive) Verkleinerung des Magenvolumens, verknüpfen.

Die Lösung des gestellten Problems gelingt bei einer gattungsgemäßen Bypass-Vorrichtung dadurch, dass das gastrische Ballonsegment und/oder das duodenale Ballonsegment das trans-pylorische Durchleitungselement radial außerhalb umgibt (umgeben), entlang einer in toroidaler Richtung umlaufenden Linie nicht geschlossen ist (sind), also nur zweifach zusammenhängend ist (sind), und derart mit dem Durchleitungselement verbunden ist (sind), dass letzteres einen Teil der Umfassung des betreffenden, toroidalen Hohlraums bildet, wobei das Durchleitungselement zumindest in seinem den gastrischen und/oder duodenalen Hohlraum umfassenden Bereich derart ausgebildet oder verstärkt ist, dass es dort eine zumindest gleich große oder höhere Strukturstabilität oder Selbstaufrichtungsfähigkeit aufweist als das betreffende Ballonsegment in der unmittelbaren Umgebung des Durchleitungselements.

Die vorliegende Erfindung beschreibt damit eine endoskopisch platzierbare, trans-pylorisch positionierte Bypass-Vorrichtung zur Durchleitung von Speisebrei aus dem Magen durch das Duodenum, welche vorzugsweise auf sehr dünnwandigen, jedoch formstabilen, komplex ausgeformten Ballonfolien beruht. Die im Folgenden beschriebenen Ausführungsformen der Erfindung gestatten es, die radiale Ausdehnung der trans-pylorisch ankernden Ballonsegmente bei Beaufschlagung mit Fülldruck gut reproduzierbar auf ein nicht zu überschreitendes Zielmaß einzustellen. Durch die bauartbedingte Generierung einer in axialer Wirkrichtung auf die Schulterflächen des pylorischen Schließmuskels hin gerichteten Rollbewegung der Ballonsegmente, kann eine besonders vorteilhafte, kombiniert radial und axial wirkende Anker- und Dichtungswirkung erreicht werden.

Bedingt durch die vollständig bzw. nahezu vollständig ausgeführte Vorformung sämtlicher Segmente der Ballonhüllen auf die für ihre Funktion in situ erforderlichen Abmessungen sowie durch die Verwendung wenig volumendehnbarer, bei Beaufschlagung mit Fülldruck weitgehend formstabiler Folienmaterialien, entwickeln die in die ankernde Vorrichtung integrierten Ballonanteile bereits bei geringfügiger Überschreitung des im Übergangsbereich von Magen und Duodenum herrschenden Binnendruckes ihre funktionell erforderliche Geometrie und mechanische Charakteristik. Die erfindungsgemäß ausgeformten Ballonkörper erfordern also zur Entfaltung ihrer Funktion keine kraftintensive elastische Aufdehnung der Ballonwandung und können so weitgehend druckneutral und damit schonend platziert werden.

Bei einer Steigerung des Fülldrucks bleibt, bedingt durch die besondere Härte des verwendeten Folienmaterials bzw. dessen geringer Volumendehnbarkeit (Compliance), die radiale Expansion der Ballonfolie begrenzt, während die applizierte Kraft vorzugsweise in eine axial gerichtete Anpresswirkung der einander axial gegenrollenden Ballon- bzw. Widerlagersegmente übergeht. Abhängig vom jeweiligen Fülldruck, kann die so auf den Pylorus anlastende axial Kraftwirkung vom Anwender über einen optimal weiten Bereich angepasst werden.

Die Erfindung beschreibt desweiteren besondere Ausführungsformen des trans-pylorischen Segmentes der Vorrichtung, welches innerhalb des Pylorus-Kanals platziert ist und das gastrische und duodenale Widerlagerelement verbindet. Hierbei steht im Vordergrund, die Schließfähigkeit des Pylorus so weit wie möglich zu erhalten und dabei axiale Verwindungen des Durchleitungssegmentes auszuschließen. Dies wird in einer bevorzugten Ausführungsform der Bypass-Vorrichtung durch eine koaxial doppellagige Schlauchfolienanordnung ermöglicht, die durch spezifisch angebrachte, punktuelle oder linienartige Verbindungen der beiden konzentrischen Folienlagen, angetrieben von der aktuell auf den Ballonelementen anlastenden Kraft, sowohl eine Lumenaufrichtung des durchleitenden pylorischen Segmentes als auch dessen axiale Entwindung bewirkt.

Zur Verbesserung des gewichtnormalisierenden Effektes, über die duodenale Bypass-Wirkung hinaus, kann die erfindungsgemäße Vorrichtung in ihrer Ausführung optional derart ausgestattet werden, dass das im Magen platzierte und dort primär als Widerlager wirkende Ballonsegment auf ein Durchmesser- bzw. Volumenmaß ausgeformt wird, welches im pylorusnahen Antrum des Magens raumfordernd wirkt und über eine moderate, aber permanent wirkende Dehnung der Wandung des Antrums ein Sättigungsgefühl auslöst bzw. bei Nahrungsaufnahme den Zeitpunkt eines Sättigungsgefühls nach vorn verlagert. Diese restriktive Wirkung kann alternativ auch durch ein zusätzliches, separat befüllbares, beispielsweise torus-artig ausgeformtes, sich magenseitig an die beschriebene trans-pylorische Ankervorrichtung anschließendes und entsprechend raumforderndes Ballonelement verstärkt werden.

Zur Erreichung einer möglichst prompten, elastisch wirkenden Lumenaufrichtung trans-pylorischen Durchleitungssegmentes wird zur Herstellung der entsprechenden Komponenten vorzugsweise Polyurethan (PUR) als Grundmaterial verwendet. Das durch den Pylorus hindurch reichende Schaftelement, welches in bevorzugter Bauweise magen- und duodenalseitig eine Ballonkomponente trägt, kann beispielsweise aus einem schlauchartigen Körper ausgeformt oder auch spritzgegossen werden. Für das ballontragende Schaftelement kommen bevorzugt Polyurethane des Härtegradbereichs Shore 70A bis 90A zur Verwendung. Die elastischen Verformungs- und Aufrichtungseigenschaften des Schaftelementes können durch zusätzliche hülsen- oder schlauchartige Elemente auf beispielsweise Schaum-, Faser-, Netz- oder Gelbasis modifiziert werden. Derartige Modifikationen können vor allem im Bereich der endständigen Anteile des durchleitenden Schaftelementes erforderlich sein, welche einen ring- oder zylinderartig gestalteten Ballon tragen. Die modifizierenden Strukturen werden entweder in das Lumen des Durchleitungselementes eingeschoben oder alternativ auf dessen äußerem Umfang verbaut. Die konzeptionell gewünschte radiale Faltbarkeit jenes Anteils des Durchleitungselementes, der unmittelbar pylorisch platziert ist, soll bei entsprechenden Modifizierungen so weit wie möglich erhalten bleiben.

Die Wandung der in die trans-pylorisch ankernde und dichtende Kopfeinheit der Vorrichtung integrierten Ballonelemente besteht ebenfalls bevorzugt aus Polyurethan. Die bevorzugten Durometer reichen von Shore 80A bis 95A einerseits sowie von Shore 55D bis 70D andererseits. Vor allem Polyurethane der Härten Shore 95A sowie 55D bis 65D gewährleisten selbst bei dünnwandigster Ausführung im niedrigen Mikrometerbereich, auch bei höheren Fülldrucken, die für die zuverlässige Funktion erforderliche Stabilität. Für die dauerhafte Platzierung im aziden Milieu des Magens sind höhere Durometer aromatischer PUR-Typen zu bevorzugen. Zur weiteren Verbesserung der Säurebeständigkeit können die PUR-basierten Ballonhüllen optional mit einer zusätalichen äußeren Lage aus PEBAX versehen sein. Die Kombination der beiden Materialien kann zum Beispiel durch eine entsprechende Ko-Extrusion des durch Blasformung zum Ballon umgeformten Rohschlauchmaterials gewährleistet werden.

Ein weiterer Vorteil der erfindungsgemäßen, extrem dünnwandigen Ausführung der ankernden und dichtenden Ballonelemente besteht in den durch sie ermöglichten geringen Baugrößen. Trotz des relativ komplexen Aufbaus der Vorrichtung können die beschriebenen folienbasierten Bypässe komfortabel endoskopisch appliziert werden. Die auf der Außenfläche des Endoskopschaftes aufgebrachte und so durch den Rachen und die Speiseröhre in den Magen des Patienten transportierte Vorrichtung trägt im evakuierten Zustand optimal schlank und die Passage nicht behindernd auf dem Endoskoprücken auf.

Die para-pylorischen Anteile der Vorrichtung sind vorzugsweise mit röntgendichten Strukturen versehen, die im Bildwandler bei Bedarf die Bestätigung der trans-pylorischen Lage erlauben.

Die befüllbaren Elemente der Vorrichtung werden in der bevorzugten Bauweise über ausreichend lang bemessene Schlauchzuleitungen von extrakorporal her befüllt. Die Zuleitungen werden im Magen deponiert und können von dort bei Bedarf endoskopisch geborgen werden.

Der trans-pylorischen Kopfeinheit schließt sich zum Duodenum hin die trans-duodenal durchleitende, den eigentlichen Bypass darstellende Schlaucheinheit an. Die duodenal durchleitende Schlaucheinheit ist platzsparend, bevorzugt in faltenbalgartiger Weise gerafft, auf das duodenalseitige Ende der trans-pylorischen Einheit aufgebracht bzw. schließt sich, entsprechend platzsparend angeordnet unmittelbar an das duodenale Ballon- oder Widerlagersegment an.

Die Bypass-Vorrichtung kann vom Anwender durch einen vorzugsweise separaten, auf dem Endoskop- oder Applikatorschaft aufgebrachten Träger- und Kopplungsmechanismus auf dem Schaft fixiert oder von diesem freigegeben werden. Eine derartige Kupplung kann beispielsweise durch einen von außen befüllbaren, hohlzylinderartigen Ballon realisiert werden, welcher zwischen dem Endoskopschaft und der Bypass-Vorrichtung wirkt und auf den Schaft des Endoskops aufgezogen wird. Er wird für die Dauer der Einführung und Anlage des Bypass mit Druck beaufschlagt und hält die Bypass-Vorrichtung so sicher in Position.

Der erfindungsgemäß optionale Aufbau der trans-pylorischen Kopfeinheit aus vorzugsweise durchgängig geformten Ballonhüllen, welche im idealen Falle sowohl Anteile des gastrischen, des duodenalen als auch des mittigen pylorischen Segmentes der Kopfeinheit umfassen, ist ferner von Vorteil, als potentiell kritische Fügestellen reduziert werden können. Die Erfindung beschreibt entsprechende Ausführungsformen, die nahezu vollständig aus einer einzigen Ballonhülle bestehen und durch entsprechende Einstülpung bzw. Rückstülpung der Ballonenden die Anzahl der zur Schaffung der verschiedenen Kompartimente innerhalb der Kopfeinheit erforderlichen Fügestellen auf ein Minimum reduzieren.

Zur Erzeugung der erfindungsgemäß optional beschriebenen, axial gerichteten Gegenrollbewegung der magenseitig und duodenalseitig wirkenden Ballon- bzw. Widerlagerkomponenten wird ein vorzugsweise zylindrisch ausgeformtes Ballonelement bei der Montage derart auf einem den Ballon tragenden Schaftelement aufgebracht, dass die Schattenden des Ballons auf dem Schaft um einen bestimmten Betrag, z.B. 30 - 60 % der frei entfalteten zylindrischen Länge des Ballons, zueinander hin gerichtet versetzt werden. Der so auf dem Schaft fixierte Ballon rollt dann bei Befüllung in eine spannungsarme Mittelposition über den beiden zueinander hin versetzten bzw. einander angenäherten Ballonenden. Aus dieser Ruhelagelage heraus kann der Ballonkorpus axial ausgelenkt werden, wobei eine der Auslenkungsrichtung entgegengesetzt wirkende axiale Kraft überwunden werden muss. Werden die Fixierungsstellen eines derartig aufgebrachten Ballonkörpers nahe genug an eine zu verschließende Öffnung positioniert, stemmt sich die zu der Körperhöhlung oder -öffnung hin gerichtete Schulter des Ballonzylinders nach Art einer axial wirkenden Rollbewegung gegen diese ab und hält das entsprechende Widerlagerelement auf der anderen Seite dieser Körperhöhlung oder -öffnung unter einer korrespondierenden, axial gegen die zu dichtende Körperhöhlung oder -öffnung gerichteten Spannung. Dieser Effekt existiert sowohl bei einseitig neben einer Körperhöhlung oder -öffnung als auch beidseitig zu einer Körperhöhlung oder öffnung angebrachten, gegenrollenden, voneinander strukturell separierten, optional separat oder kommunizierend befüllbaren Ballonelementen.

Weiterhin gibt es eine taillierte Ausführungsform eines ausgeformten Ballonkörpers, welcher zwischen zwei endständigen, dichtend wirkenden Ballonsegmenten einen mittigen, verjüngten Abschnitt aufweist, der nicht mit dem ballontragenden Schaft verbunden ist und innerhalb der Sphinkteröffnung platziert wird, wobei sich die endständigen Ballonsegmente bei entsprechendem, zu einander hin gerichteten Versatz der Ballonenden auf dem Schaft ebenfalls durch eine von lateral zur mittigen Taille hin gerichtete, klemmend und dichtend wirkende Rollbewegung auszeichnen.

Die vorliegende Erfindung beschreibt ferner einen Selbstaufrichtungsmechanismus koaxial angelegter, durch punktuelle Verbindungen verbundener Schlauchfolienlagen. Dabei kann sich ein Teil des Füllmediums in den freien Raum zwischen den konzentrischen Folienlagen verschieben. Dieser Abschnitt richtet sich dann nach Art eines matratzenartig abgesteppten, sich selbst aufrichtenden, luftstabilisierten Rohrkörpers auf. Bei nachlassendem Fülldruck entspannt sich dieser Teil der Vorrichtung, und das Durchleitungslumen kann sich unter der anlastenden Kraft des sich schließenden Pylorus zu einer minimal raumfordernden, nicht dilatierenden Struktur zusammenziehen.

In einer besonderen Ausführungsform kombiniert die vorliegende Erfindung das Prinzip der axial gerichteten Gegenrollbewegung mit Wirkungsweise der konzentrischen, sich bei Beaufschlagung mit Druck selbstaufrichtenden Folienanordnung.

Bevorzugt umfasst sowohl das magenseitige als auch das duodenalseitige Widerlagerelement ein derartiges, befüllbares Ballonsegment. Das mittige, trans-pylorische Segment kann den beschriebenen, konzentrisch angeordneten Folienaufbau aufweisen und ist bevorzugt mit beiden Widerlagerballonsegementen, bzw. - weniger bevorzugt - nur mit dem magenseitigen Ballonkompartiment frei kommunizierend verbunden.

In einer besonders vorteilhaften Ausführung werden alle drei Segmente aus einem einzigen Formrohling ausgeformt und anschließend durch partielle oder auch vollständige Rückstülpung bzw. Inversion der Ballonenden zu einem einzigen, kommunizierenden Raum geschlossen oder auch, im Verbund mit stabilisierenden Schlauch- oder Ringkomponenten, zu mehreren, kommunizierenden Teilräumen kompartimentiert. Durch eine punktuelle oder linien- bzw. stegartige Verbindung der konzentrischen Folienlagen im dem Schließmuskel exponierten, trans-pylorischen Segment, erreicht dieses seine lumen-aufrichtende bzw. lumen-entwindende Wirkung.

Neben der bevorzugten Ausführung des trans-pylorischen Segmentes der Vorrichtung als selbstaufrichtende, konzentrische Folienanordnung, kann der trans-pylorische Anteil beispielsweise auch als durchgehendes Rohrelement ausgebildet sein, welches derartig elastisch wirkt, dass es unter Kontraktion des Pylorus zu einer Struktur kleineren Durchmessers kollabiert und sich bei nachlassendem Tonus in den sich öffnenden Pylorus spontan hinein aufrichtet. Die elastische Selbstaufrichtung des Rohrelementes muss dabei dem Druck, der im Ballon auf das Rohrelement wirkt, wiederstehen und eine Einengung des Drainagelumens ausschließen. Zur Optimierung der selbstaufrichtenden Eigenschaften, kann der Rohrquerschnittes mit einer stabilisierenden ring- oder wendelartigen Korrugation versehen sein, welche bei gleicher elastischer Rückstellkraft, eine für die Platzierung vorteilhafte Reduktion der Wandungsstärke des Elementes erlaubt. Neben der beschriebenen Korrugation des Rohrelementes, kann das Rohr auch als kombinierte Struktur eines durchgängigen Rohrschaftes, mit einer den Schaft stabilisierenden, ummantelnden oder auch auskleidenden Materiallage, beispielsweise netzartiger, faser- oder schaumartiger Beschaffenheit bestehen. Die so mögliche Modifikation der elastischen Wirkkomponenten der einzelnen Materiallagen gewährleistet eine ideale Einstellbarkeit der Verschluss- und Öffnungskraft, mit der sich der trans-pylorische Rohrkörper bei einer Kontraktion des Sphinkters verschließet bzw. sich bei sich öffnendem Sphinkter in diesen hineinentwickelt.

Wenigstens ein Ballonsegment sollte keinen vollkommen geschlossenen Torus bilden, sondern wenigstens eine, rundum laufende, ringförmig geschlossene, dem trans-pylorischen Durchleitungselement anliegende, freie Kante aufweisen. Mittels jener freien Kante lässt sich das betreffende Ballonsegment präzise auf dem Durchleitungslumen positionieren, und im Zusammenwirken mit seiner Präformierung kann sein Verhalten beim Befüllen genau beeinflusst werden.

Die wenigstens eine rundum laufende, ringförmig geschlossene, freie Kante wenigstens eines Ballonsegmentes lässt sich mit dem trans-pylorischen Durchleitungselement dichtend verbinden, insbesondere verkleben oder verschweißen.

Wenigstens ein Ballonsegment kann im Bereich wenigstens einer, mit dem trans-pylorischen Durchleitungselement dichtend verbundenen, freien Kante derart (mehrfach) umgestülpt sein, dass es mit seiner dem betreffenden Hohlraum zugewandten Innenseite an dem trans-pylorischen Durchleitungselement flächig anliegt.

Wenigstens ein Ballonsegment kann im Bereich seiner beiden freien Stirnkanten jeweils nach innen in das betreffende Ballonsegment hinein umgestülpt sein.

Bevorzugt liegt zwischen einer freien Stirnkante und deren Umstülpung in das betreffende Ballonsegment hinein eine zweite, entgegengesetzte Umstülpung, also in Richtung aus dem betreffenden Ballonsegment heraus.

Weitere Vorteile ergeben sich dadurch, dass ein Ballonsegment derart präformiert ist, dass es in bestimmten ringförmigen Abschnitten unterschiedliche Umfangslängen aufweist, insbesondere dass es im Bereich seiner beiden freien Stirnkanten jeweils eine kleiner Umfangslänge aufweist als in einem dazwischen liegenden Bereich des Ballonmantels, der einen sich nach außen stülpenden Abschnitt des Ballonsegments bildet. Dadurch kann die Form des von äußeren Kräften freien, entfalteten Ballonsegments ebenso vorgegeben bzw. beeinflusst werden wie dadurch, dass ein Ballonsegment derart präformiert ist, dass es in bestimmten ringförmigen Abschnitten unterschiedliche Stärken aufweist, insbesondere dass es im Bereich seiner beiden freien Stirnkanten jeweils eine größere Stärke aufweist als in einem dazwischen liegenden Bereich des Ballonmantels, der einen sich nach außen stülpenden Abschnitt des Ballonsegments bildet.

Indem ein Ballonsegment derart präformiert ist, dass bei bis zu seinem vorgeformten Volumen expandiertem Hohlraum der Querschnitt durch diesen Hohlraum eine größere Axialerstreckung aufweist als in radialer Richtung, bezogen auf die Längsachse des Durchleitungselements, ist eine überwiegende Ersteckung des entfalteten Ballonsegments in axialer Richtung vorgegeben. Daraus resultiert eine optimale Fähigkeit, einerseits in axialer Richtung zurück rollen zu können, um Platz für den Pylorus zu schaffen, aber auch eine entgegen dieser Ausweichbewegung gerichtete Anpresskraft zu entwickeln, um dadurch das Durchleitungselement zu verankern. Dabei wirkt die Dichtungskraft fokussiert auf die Schulterflächen des pylorischen Schließmuskelringes, wo dann ein Formschluss ausgebildet werden kann, der einem Herausschlüpfen des Durchleitungselements aus dem Bereich des Pylorus entgegenwirkt.

Demzufolge ist die Kraftexposition auf die dem Pylorus angrenzenden Strukturen des Magens und des Duodenums, auch bei einem höherem Fülldruck der Ballonsegmente, auf ein dauerhaft organverträgliches Maß reduziert, insbesondere auf einen Druck unterhalb des Fülldruckes der Ballonsegmente.

Der Füllzustand der Ballonsegmente und/oder deren Fülldruck, und damit die axial auf den Pylorus wirkende Dichtungskraft sollte von extrakorporal her einstellbar sein, ggf. unter Zuhilfenahme einer Pumpe und/oder eines Manometers.

Als Fülldruck innerhalb eines oder beider Ballonsegmente sieht die Erfindung Werte von 10 mbar bis 100 mbar oberhalb des atmosphärischen Drucks vor, insbesondere auf Werte von 20 mbar bis 80 mbar oberhalb des atmosphärischen Drucks, insbesondere auf Werte von 30 mbar bis 60 mbar oberhalb des atmosphärischen Drucks.

Die Erfindung lässt sich dahingehend weiterbilden, dass innerhalb wenigstens eines, von einem Ballonsegment bereichsweise umgrenzten Hohlraums ein zusätzliches, inneres Kissen oder Ballonelement angeordnet ist, welches mit einem anderen Füllmedium befüllt oder befüllbar ist als der jenes aufnehmende Hohlraum selbst.

Ein solches, inneres Kissen oder Ballonelement sollte auf dem trans-pylorischen Durchleitungselement rundum dichtend fixiert sein, insbesondere verschweißt oder verklebt, so dass es nicht verrutschen kann.

Bevorzugt umschließt wenigstens ein inneres Kissen oder Ballonelement ein kleineres Volumen und/oder ist mit einem kleineren Volumen präformiert als das von dem jenes aufnehmenden Hohlraum abgestützte, außenliegende Ballonsegment, so dass innerhalb des äußeren Ballonsegments neben dem inneren Kissen oder Ballonelement noch ein (Rest-) Hohlraum verbleibt, der vorzugsweise mit einem kompressiblen Medium wie Luft oder Gas befüllbar ist und sich dann optimal und vorzugsweise dichtend der Innenseite des betreffenden Organs anschmiegen kann.

Es hat sich bewährt, dass wenigstens ein inneres Ballonelement mit einem flüssigen Medium befüllbar ist. Dadurch ergibt sich - in Verbindung mit einem vergleichsweise harten Ballonmaterial wie bspw. Polyurethan - eine maximale Strukturstabilität, wodurch ein unerwünschtes Lösen eines solchen Verankerungsteils von der vorgesehenen Verankerungsstelle nahezu ausgeschlossen ist. Der äußere Ballon sollte daher mit Luft befüllt sein, also einem kompressiblen Medium, um sich der Innenseite des betreffenden Organs optimal anschmiegen und jenes abdichten zu können.

Das Durchleitungselement erfährt im Bereich wenigstens eines inneren Ballonelements eine Verstärkung durch eine Hülse oder ein vorzugsweise ring- oder spiralförmiges Federelement. Solchenfalls ist es in der Lage, ohne Verengung des Durchleitungslumens auch vollständig entfaltete Verankerungselemente abstützen zu können, ohne zu kollabieren.

Die Erfindung empfiehlt, dass wenigstens ein inneres Ballonelement torusförmig ausgebildet ist, also dreifach zusammenhängend. Dadurch erhöht sich seine Strukturstabilität, und ein das Durchleitungslumen verengende Effekt bei Befüllung des betreffenden, inneren Ballonelements wird minimiert.

Wenigstens ein inneres Ballonelement sollte im Bereich einer oder beider seiner ringförmig umlaufenden Stirnkanten mit seiner Innenseite, welche seinem Füllmedium zugewandt ist, auf dem trans-pylorischen Durchleitungselement flächig anliegen und dort fixiert sein, beispielsweise angeklebt.

Die Erfindung lässt sich dahingehend weiterentwickeln, dass das gastrische Ballonsegment und das duodenale Ballonsegment zu einem einzigen Ballon vereinigt sind, der hantelförmig präformiert ist, mit einer etwa mittigen, rundum laufenden Verjüngung zur Aufnahme des Pylorus-Schließmuskels. Damit kann die Gestalt des Pylorus nach Art einer Negativ-Form optimal nachempfunden werden.

Die Erfindung zeichnet sich weiterhin aus durch wenigstens eine Zuleitung oder einen Befüllschlauch zu wenigstens einem toroidalen Hohlraum, so dass ein oder beide Ballonsegmente nach der Platzierung der trans-pylorischen Bypass-Vorrichtung, insbesondere des trans-pylonischen Durchleitungselements und/oder der Fixiereinheit, befüllbar sind. Damit das orale oder proximale Ende einer solchen Zuleitung oder eines solchen Befüllschlauches in den Magen einführbar und/oder dort abwerfbar ist, sollte wenigstens eine Zuleitung zu wenigstens einem toroidalen Hohlraum, insbesondere ein Befüllschlauch, mit einem Rückschlagventil versehen sehen.

Indem das trans-pylorische Durchleitungselement das gastrische Ankerelement mit dem duodenalen Ankerelement verbindet, erfährt die Anordnung jedenfalls in axialer Richtung eine optimale Strukturstabilität.

Die Wandstärke des trans-pylorischen Durchleitungselements sollte jedenfalls in seinem den Pylorus durchsetzenden Kopfbereich dicker sein als die Wandstärke des gastrischen Ballonsegments an dessen nach radial außen gewölbter Peripherie und/oder als die Wandstärke des duodenalen Ballonsegments an dessen nach radial außen gewölbter Peripherie, beispielsweise wenigstens doppelt so dick, vorzugsweise wenigstens 5 mal so dick, insbesondere wenigstens 10 mal so dick. Dadurch wird die gegenseitige Lage der beiden Ankerelemente vorgegeben.

Das trans-pylorische Durchleitungselement kann schlauchförmig präformiert sein, so dass es bei Befüllung eines oder beider Ballonsegmente unter dem dortigen Fülldruck nicht kollabiert, sondern sich in von äußeren Kräften freiem Zustand von selbst in eine etwa zylindrische Form aufrichtet.

Indem das trans-pylorische Durchleitungselement gewellt oder korrugiert ist, so werden seine selbstaufrichtenden Eigenschaften verbessert, und außerdem lässt es sich in faltenbalgartiger Weise zusammenschieben oder raffen.

Das trans-pylorische Durchleitungselement kann im Bereich eines oder beider Ballonsegmente durch eine Hülse oder eine ring- oder spiralförmige Feder versteift sein, so dass es bei Befüllung eines oder beider Ballonsegmente unter dem dortigen Fülldruck nicht kollabiert.

Ferner besteht die Möglichkeit, dass das trans-pylorische Durchleitungselement eine koaxial doppellagige Schlauchfolienanordnung aufweist, oder dass es aus einer solchen, koaxial doppellagigen Schlauchfolienanordnung besteht.

Die beiden Schlauchfolienlagen eines solchen, doppellagig aufgebauten, trans-pylorischen Durchleitungselements sollten zumindest bereichsweise miteinander verbunden sein, vorzugsweise durch punktuelle, linienartige oder flächige Verbindungen, insbesondere in dem den Pylorus durchsetzenden Abschnitt. Durch einen (geringen) inneren Überdruck zwischen diesen beiden Schlauchlagen wird die äußere Lage nach außen gedrückt und nimmt dabei aufgrund der Verbindungen die innere Schlauchlage mit. Das Durchleitungslumen ist daher von Natur aus offen, kann aber nötigenfalls von dem Pylorus aufgrund des geringen Fülldrucks leicht zusammengedrückt werden.

Herstellungsbedingte Vorteile können sich ergeben, indem die Schlauchfolienlagen des trans-pylorischen Durchleitungselements zusammen mit einem oder beiden Ballonsegmenten aus einem gemeinsamen Folienschlauch geformt sind.

Wenigstens eine an dem trans-pylorischen Durchleitungselement und/oder in oder an einem oder beiden Fixierelementen proximal und/oder distal des Pylorus platzierte, röntgendichte Markierung, dient der Bestimmung der korrekten trans-pylorischen Lage der trans-pylorischen Bypass-Vorrichtung, insbesondere des trans-pylonischen Durchleitungselements und/oder dem (der) Fixierelement(e).

Zur Ptatzierung einer erfindungsgemäßen trans-duodenalen Bypass-Vorrichtung im Bereich des Pylorus eines Patienten dient ein Applikator, der sich durch ein Applikator-Element in Form eines Endoskops oder Katheters auszeichnet, mit einem langgestreckten Schaft, auf dessen Außenfläche die Bypass-Vorrichtung derart aufsetzbar und/oder aufsteckbar ist, dass der Applikatorschaft das zentrale Durchleitungslumen der Bypass-Vorrichtung ganz oder teilweise durchsetzt.

Die Erfindung empfiehlt, dass dabei die Bypass-Vorrichtung auf dem Applikatorschaft reversibel, d.h. lösbar, gehalten ist.

Vorzugsweise ist die Bypass-Vorrichtung auf dem Applikatorschaft klemmend gehalten, wobei zur klemmenden Fixierung der Bypass-Vorrichtung an der Außenseite des Applikatorschaftes ein ringförmiger, von extrakorporal befüllbarer, spaltüberbrückender Ballon vorgesehen ist, welcher sich bei aufgesetzter oder aufgesteckter Bypass-Vorrichtung in dem ringförmigen Spalt zwischen dem Applikatorschaft einerseits und dem trans-pylorischen Durchleitungselements andererseits befindet, insbesondere radial innerhalb der Fixiereinheit und/oder radial innerhalb des vorderen Endes des trans-duodenal durchleitenden Schlauches.

Ein Ventil, vorzugsweise ein manuell zu öffnendes Rückschlagventil, erlaubt das Be- und Entlüften des die Bypass-Vorrichtung auf dem Applikatorschaft festklemmenden Ballons.

Ein Applikator zeichnet sich bevorzugt ferner aus durch Spülöffnungen, vorzugsweise distal des spaltüberbrückenden Ballons, durch welche über eine längs des Applikators verlaufende Spülleitung von extrakorporal Flüssigkeit in das Duodenum einspülbar ist, um den distalen Abschnitt des Durchleitungselements zu entfalten.

Eine erfindungsgemäße Bypass-Vorrichtung lässt sich unter Verwendung eines Applikators wie folgt im Pylorus eines Patienten platzieren:
a) Aufsetzen oder Aufstecken der Bypass-Vorrichtung auf den Applikatorschaft, so dass dieser das zentrale Durchleitungslumen durchsetzt;
b) Fixieren der Bypass-Vorrichtung durch Befüllen eines spaltüberbrückenden Ballons zwischen Apptikatorschaft und Innenseite des Durchleitungslumens;
c) Einführen der Bypass-Vorrichtung mittels des Applikators in den Magen eines Patienten;
d) teilweises oder vollständiges Befüllen des gastrischen Ballonsegments des gastrischen Ankerelements;
e) Einführen des distalen Abschnitts des Durchleitungselements durch den Pylorus, bis das ganz oder teilweise befüllte, gastrische Ballonsegment proximal des Pylorus aufliegt und sich einem weiteren Voranschieben der Bypass-Vorrichtung spürbar widersetzt;
f) Befüllen des duodenalen Ballonsegments zur Verankerung des duodenalen Ankerelements distal des Pylorus;
g) Freigeben des distalen Abschnitts des Durchleitungselements;
h) Lösen der Bypass-Vorrichtung durch Entlüften des spaltüberbrückenden Ballons zwischen Applikatorschaft und Innenseite des Durchleitungslumens.

Dabei kann zwischen den Schritten g) und h) über distal des spaltüberbrückenden Ballons an dem Applikatorschaft angeordnete Spülöffnungen von extrakorporal Flüssigkeit in das Duodenum gespült wird, um den distalen Abschnitt des Durchleitungselements zu entfalten.

Schließlich entspricht es der Lehre der Erfindung, dass nach dem Schritt h) die Zuleitungen zu den Ballonsegmenten in den Magen abgeworfen werden und dort verbleiben, bis die Bypass-Vorrichtung entfernt wird.

Weitere Merkmale, Eigenschaften, Vorteile und Wirkungen auf der Basis der Erfindung ergeben sich aus der folgenden Beschreibung bevorzugter Ausführungsformen der Erfindung sowie anhand der Zeichnung. Hierbei zeigt:
- Fig. 1: eine Ausführungsform mit einseitig aufgebrachter, rückrollender Ballonkomponente und gegenüberliegendem, nicht-rückrollenden Ballonelement;
- Fig. 1a: die Konfiguration des in Fig. 1 dargestellten, rückrollenden Ballonelementes der die Vorrichtung fixierenden Kopfeinheit, im frei entfalteten Zustand außerhalb des Körpers;
- Fig. 1b: die zuvor dargestellte Kopfeinheit in situ und verbildlicht die axial orientierte Rückroll- bzw. Gegenrollbewegung der Ballonkomponente zum Pylorus hin;
- Fig. 1c: eine wellschlauch-artig korrugierte Ausführungsform des im Pylorus platzierten, trans-pylorischen Durchleitungselementes;
- Fig. 2: eine Ausführungsform mit beidseitig angeordneten, axial zum Pylorus hin rollenden Ballonkomponenten;
- Fig. 2a: eine der Fig. 2 entsprechende Ausführungsform mit beidseitig rückrollenden Ballonkomponenten, wobei der magenseitige Ballon raumfordernd wirkendem, vergrößert ist;
- Fig. 3: eine Ausführungsform der Erfindung mit beidseitig angeordneten, rückrollenden Ballonkomponenten, im Verbund mit einem konzentrisch doppellagigen, folienbasierten trans-pylorischen Durchleitungselement;
- Fig. 3a: die steg-artige Verbindung der konzentrischen Folienlagen im Bereich der trans-pylorischen Durchleitung;
- Fig. 3b: eine Ausführungsform wie in Fig. 3, wobei jedoch die lumenstabilisierenden Hülsenelemente im inneren Lumen des Durchleitungskanals angeordnet sind;
- Fig. 3c: eine Ausführungsform wie in Fig. 3, wobei die lumenstabilisierenden Hülsenelemente im Binnenraum eines in sich rückgestülpten Ballonelementes angeordnet sind;
- Fig. 4: eine Ausführungsform mit einem durchgängig ausgeformten, im Bereich des trans-pylorischen Anteils hantelförmig verjüngten Ballonkörper;
- Fig. 5: eine Ausführungsform mit beidseitig aufgebrachten rückrollenden Ballonkomponenten, trans-duodenalem Fortleitungsschlauch und intragastrisch raumfordernden Ballonelement;
- F. 6a,b: Ausführungsformen mit einer Ballon-im-Ballon-Konfiguration und jeweils separierter Befüllung mit kompressiblen und nicht-kompressiblen Medien;
- F. 7a,b: bevorzugte Ausführungsformen mit durchgängigem, hantelförmigem, transpylorischem Dichtungsballon und einem duodenalseitig, vom Dichtungsballon partiell oder vollständig umschlossenen, zusätzlichen Fixierungsballon;
- F. 8a,b: an Fig. 7a und 7b angelehnte Ausführungsformen, wobei das zusätzliche fixierende Element auf beiden Seiten des Pylorus angeordnet ist;

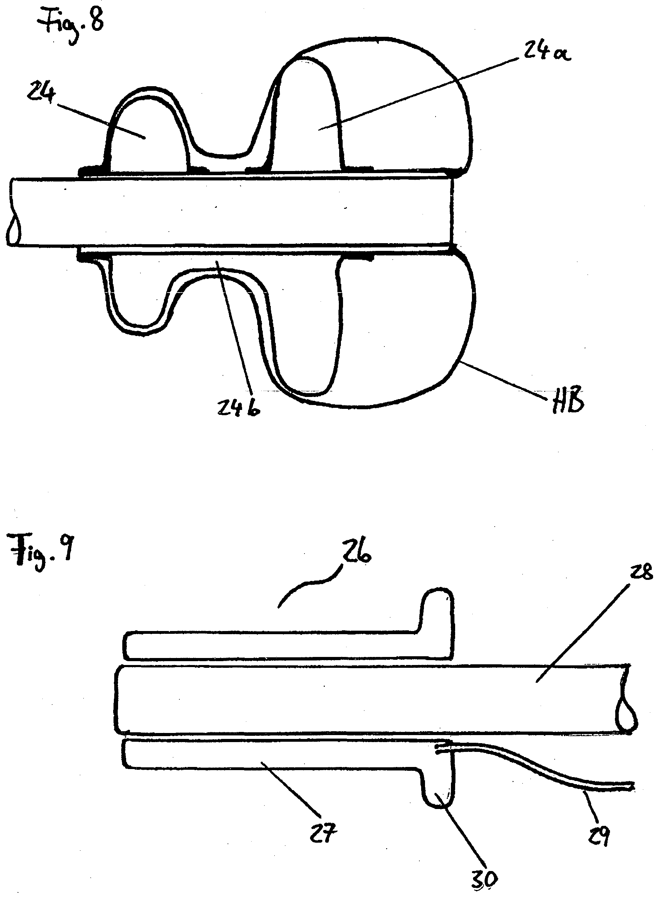

- Fig. 9a: eine einfache Variante einer Transport-, bzw. Fixier- oder Abwurfvorrichtung für die Applikation der Vorrichtung auf dem Endoskoprücken;
- Fig. 9b: eine Variante einer der Fig. 9a entsprechenden Einheit mit einer proximalen Anschlagfunktion auf der Basis einer sich ausbildenden Ballonschulter

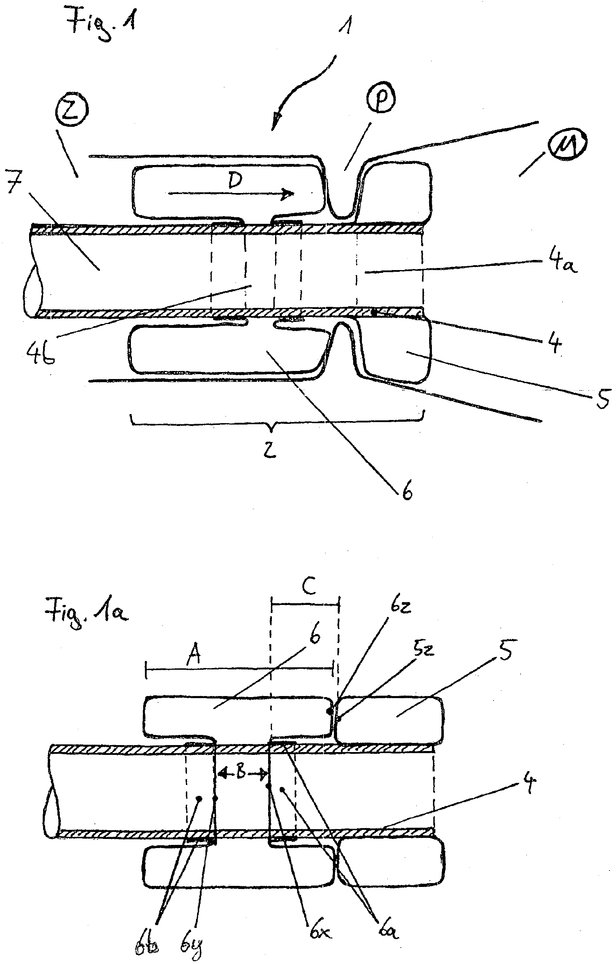

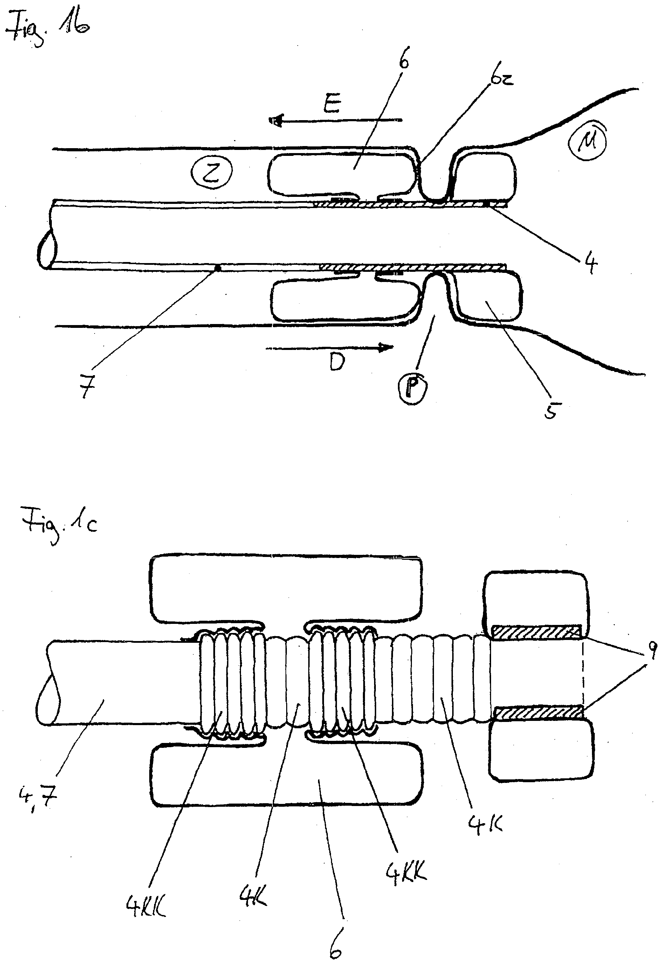

Fig. 1 stellt schematisch eine erfindungsgemäße Bypass-Vorrichtung 1 dar und beschreibt beispielhaft die erforderlichen Komponenten und baulichen Merkmale zur Generierung einer zum Pylorus P hin gerichteten Rollbewegung D eines befüllbaren Ballonelementes.

Abgebildet ist die über den Pylorus hinweg (trans-pylorisch) platzierte Fixiereinheit 2 der Bypass-Vorrichtung, welche den Nahrungsbrei aus dem Magen M aufnimmt und durch den Pylorus P in das Duodenum Z führt. Die den Bypass in seiner trans-pylorischen Position fixierende Einheit weist im Zentrum ein Durchleitungselement 4 auf, welches auf seinem magenseitigen Ende 4a ein ankerndes Widerlagerelement 5 trägt und an seinem duodenalseitigen Ende 4b mit einem, im befüllten Zustand, zum Widerlagerelement hin rollenden, in spezifischer Weise ausgeformten und auf dem Durchleitungselement fixierten, duodenalen Ballonelement 6 ausgestattet ist. An das duodenal-seitige Ende 4b des Durchleitungselementes schließt sich ein Schlauchelement 7 an, das den Nahrungsbrei durch das Duodenum hindurch leitet.

Fig. 1a zeigt die in Fig. 1 beschriebene trans-pylorische Fixierungseinheit 2 im befüllten Zustand außerhalb des Körpers. Das gegenrollende duodenalseitige Ballonelement 6 ist hier in einer vorzugsweise zylindrisch ausgeformten Form dargestellt, wobei das verwendete Ballonmaterial derart beschaffen ist, dass es bei einer Befüllung in einem Druckbereich von ca. 20 bis ca. 100 mbar, aus dem vorgeformten Zustand eine radiale Dehnung des zylindrischen Durchmessers von nicht mehr als 10%, und besonders bevorzugt von nicht mehr als 5 % durchläuft. Der ausgeformte zylindrische Durchmesser des duodenalen Ballonelementes 6 soll vorzugsweise so bemessen sein, dass er die Wandung des sich dem Pylorus anschließenden Duodenums nicht dilatiert bzw. diese nur unter eine moderate, durch die beschriebenen Dehnungseigenschaften des Ballons begrenzte Dehnung bzw. Spannung nimmt. Die sich im Falle einer Dehnung der Duodenalwand im Ballon 6 einstellenden Fülldrucke sollten 50 mbar nicht überschreiten.

Die Länge der zylindrischen Kontaktfläche A sollte bevorzugt ca. 1 bis 4 cm und besonders bevorzugt 2 bis 3 cm betragen. Die Distanz B zwischen den Ballonenden 6a und 6b sollte bevorzugt 20 bis 60 % und besonders bevorzugt 20 bis 40 % der Länge der Kontaktfläche A betragen. Für den sich ergebenden axialen Rollweg des auf dem Durchleitungselement 4 montierten Ballons sind die zum Balloninneren hin gerichteten Abschlusskanten 6x und 6y der Ballonenden ausschlaggebend. Die Distanz B wird entsprechend als Abstand zwischen den Abschlusskanten 6x und 6y definiert.

Die Fixierung des Ballons auf dem Durchleitungselement 4 sollte bevorzugt derart erfolgen, dass die Abschlusskante 6x um den Betrag der Strecke C von der pylorusseitigen Schulter 5z des gastrischen Widerlagerelementes 5 beabstandet ist, wobei C bevorzugt nicht größer sein sollte, als der Betrag, der sich aus A/2 - B/2 + 5 mm ergibt. Dieser Betrag repräsentiert einen Zustand des befüllten Ballons 6, bei dem sich der Ballon im mittigen Neutralzustand über den Abschlusskanten 6x und 6y positioniert und der sich ergebende Spalt zwischen der pylorusseitigen Schulter 6z des duodenalen Ballonelementes 6 und der magenseitigen Schulter 5z des gastrischen Widerlagerelementes eine Breite von 5 mm aufweist, was in etwa der anatomischen Breite des pylorischen Schließmuskels entspricht. In besonders bevorzugter Ausführung weist die Strecke C einen Betrag auf, der kleiner ist als A/2 - B/2 + 5 mm ist, bis zu mm reichen kann, oder auch einen Betrag aufweist, der kleiner ist als A/2 - B/2.

Fig. 1b zeigt im Schema, wie sich die duodenalseitige Schulter 6z des Ballons 6 in axial gerichteter Gegenrollung (Pfeil D) an die duodenalseitige Schulterfläche des Pylorus P anpresst. Die in Summe von der Duodenalseite Z und von der Magenseite M auf den Pylorus axial auf den Pylorus wirkenden Kraft kann durch den Anwender durch den jeweiligen Fülldruck im Ballonelement 6 eingestellt und beim Individuum im Verlauf der Anwendung bei Bedarf angepasst werden. Anders als bei bekannten duodenalen Bypass-Vorrichtungen, folgt der durch die axial federnde Rollbewegung in Richtung D zum Pylorus hin (Pfeil D) und in Gegenrichtung E vom Pylorus (Pfeil E) gewährleistete Anpressmechanismus dynamisch dem jeweiligen Funktionszustand des Schließmuskels. Funktionelle Schwankungen der Breite des Schließmuskels bzw. der Länge des Pyloruskanals werden so fortlaufend kompensiert und somit eine optimal motilitätskompatible Dichtung der Fixierungseinheit im Pylorus ermöglicht.

Bei den in den Fig. 1 und 1a bis 1c beschriebenen Vorrichtungen kann das magenseitige Widerlagerelement 5 als weiche, elastisch verformbare, beispielsweise gelartige Struktur ausgeführt sein. Alternativ kann auch ein in konventioneller Weise aufgebrachter Ballonkörper, also ohne invertierte Fixierung der Ballonenden und ohne entsprechenden Gegenrolleffekt, als gastrisches Widerlagerelement dienen.

Das Durchleitungselement 4 kann als relativ starres, rohrartiges Element ausgeführt sein, weist jedoch bevorzugt die Fähigkeit zur elastischen radialen Einfaltung und Selbstaufrichtung auf. Vorzugsweise soll sich das im Sphinkter jeweilig einstellende Lumen des Durchleitungselementes dem physiologischen Sphinkterschluss mit möglichst geringem, entgegenwirkendem, elastischen Widerstand folgen. Bei maximalem Sphinktertonus soll sich das Durchleitungselement dem vollständigen Kontraktionsweg des Sphinkters folgend, bis auf ein nahezu dicht schließendes Restlumen verformen.

Die beschriebene elastisch wirkende, radiale Faltung/Einstülpung des den Sphinkter passierenden Durchleitungselementes wird bevorzugt durch ein Rohrmaterial mit primär elastischen Eigenschaften, wie zum Beispiel Polyurethan (PUR) gewährleistet. Eine entsprechende Elastizität weisen beispielsweise PUR-Typen der Sorte Elastollan 1180A und 1185A, der Fa. BASF auf, wenn diese zu einem Rohrkörper mit einem Durchmesser von ca. 20 mm und einer Rohrwandungsstärke von ca. 200 µm ausgeformt werden.

Die elastische Faltbarkeit bzw. elastisch wirkende Aufrichtung des Rohrkörpers kann durch ein wellschlauchartiges Profil des Durchleitungselementes in ihrer Wirksamkeit verbessert werden. Ein derartiges Profil ermöglicht zudem eine Reduktion der Rohrwandungsstärke. So kann beispielsweise, wie in Fig. 1c dargestellt, ein Durchleitungselement 4 als durchgehender Rohrkörper mit einem ring- oder spiralartig gewellten (korrugierten) Profil 4k oder auch 4kk versehen sein, wobei der Rohrkörper beispielsweise aus Elastollan der Sorte 1180A besteht, einen inneren Durchmesser von ca. 20 mm aufweist, eine Wandungsstärke von ca. 150 bis 200 µm, die Korrugation eine Amplitude von 1 bis 2 mm (bevorzugt 1,5 mm) und eine peak-to-peak-Distanz von 1 bis 2,5 mm (bevorzugt 1,5 bis 2 mm) aufweist. Das korrugierte Profil kann zur weiteren Modifikation seiner elastischen Eigenschaften zudem in einzelnen Segmenten mit einer zusätzlichen, die Faltungsmechanik modifizierenden, z.B. gel- artigen Materiallage überzogen sein.

Von besonderer Bedeutung für die Funktion der Vorrichtung ist die bauliche Ausführung der endständigen Segmente 4a und 4b des Durchleitungselementes, die die Ballon- bzw. Widerlagerkörper auf ihrer Außenseite tragend aufnehmen. Die Segmente sind bevorzugt derart gestaltet, dass sie sich bei einer gewissen Krafteinwirkung elastisch einfalten und bei nachlassender Kraft entsprechend elastisch aufrichten. Prinzipiell können bei der Entwicklung einer elastisch selbstaufrichtenden Wirkung der endständigen Segmente 4a und 4b die gleichen, die Verformungs- und Aufrichtungseigenschaften modifizierenden Gestaltungselemente zur Anwendung kommen, wie zuvor beim Aufbau des trans-pylorischen Segmentes beschrieben.

Da die auf die endständigen Segmente 4a und 4b radial wirkenden Kräfte in der Regel größer sind, als die auf das trans-pylorische Segment wirkenden Kräfte, sollte deren elastische, lumenaufrichtende Wirkung entsprechend verstärkt werden, beispielsweise durch eine besonders enge Korrugation 4kk (peak-to-peak-Distanz von beispielweise kleiner 1,5 mm) oder durch das Durchleitungselement verstärkende ringförmige Elemente 9. Die elastische Wirkung der endständigen Segmente sollte derart bemessen sein, dass diese einem von außen anlastenden Fülldruck von 20 bis 100 mbar, bevorzugt 20 bis 60 mbar ohne Kollaps des Lumens Stand halten. Eine starrwandige, nicht kollabierbare Ausführung der Segmente 4a und 4b ist im Rahmen der Erfindung vorstellbar, für die Funktion jedoch nachteilig.

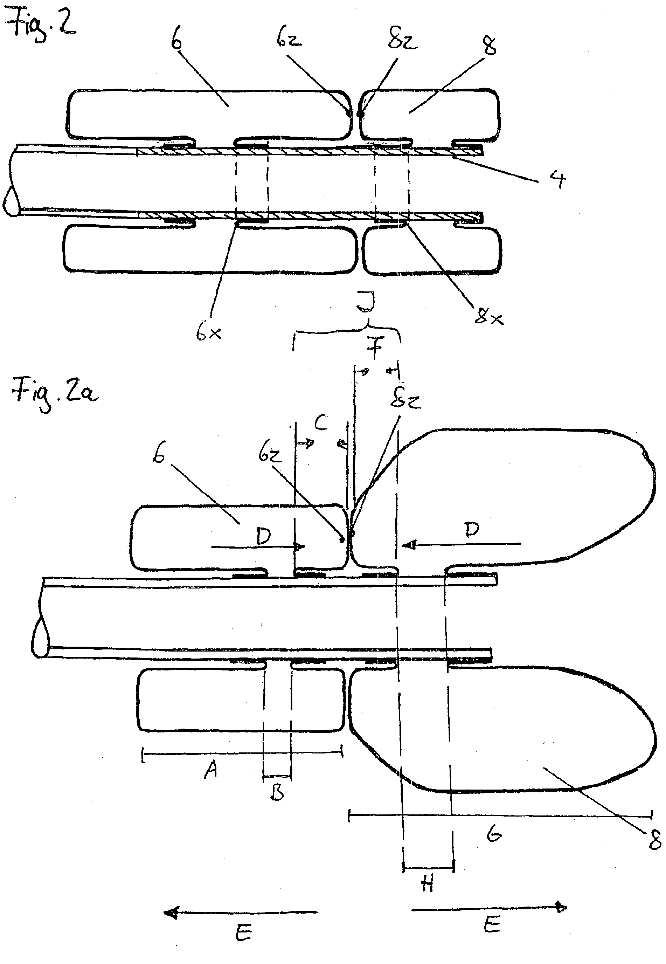

Fig. 2 und 2a zeigen die erfindungsgemäße Vorrichtung mit zwei endständig auf dem Durchleitungselement 4 angeordneten, jeweils zur Rück- bzw. Gegenrollung befähigten Ballonelementen, wobei die Ausformungsgeometrie, die Ballonmaterialien und die spezifisch invertierte Montage der Ballons auf dem Durchleitungselement bei beiden Ballonelementen den Vorschriften der vorigen Figuren entsprechen. Im bevorzugten Ausführungsfall sind die beiden einander gegenüber positionierten Ballons durch eine kommunizierende Befülleitung verbunden, es stellen sich also bei der Befüllung der Vorrichtung in beiden Kompartimenten identische Drucke ein.

Die vorzugsweise zylindrisch, mit jeweils steilen Schultern ausgeformten Ballonkörper 6 und 8 sollen dabei derart auf dem Durchleitungselement 4 platziert sein, dass sich die jeweiligen Schulterflächen 6z und 8z bei freier Befüllung der Ballone, außerhalb des Körpers, in ihrer jeweiligen Neutrallage bzw. ohne Auslenkung aus ihrer Neutrallage in einem freien Abstand von nicht mehr als 5mm, also der angenommenen Breite des Pylorus, gegenüberstehen. In diesem Falle entspricht die für die Montage der Ballonkörper maßgebende Strecke J zwischen den Abschlusskanten 6x und 8x der Summe aus (A/2 - B/2) + (G/2 - H/2) + 5 mm. Im befüllten Zustand der Ballonkörper 6 und 8 lasten die Schulterflächen 6z und 8z dem Pylorus in situ dann nahezu ohne Anpressdruck an. In den bevorzugten Ausführungsvarianten der Vorrichtung ist die montagerelevante Strecke J jedoch kleiner als die Summe aus (A/2 - B/2) + (G/2 - H/2) + 5 mm und besonders bevorzugt kleiner als die Summe aus (A/2 - B/2) + (G/2 - H/2). Die sich bei Befüllung der Ballonkörper von den einander gegenwirkenden Kräften einstellenden "Rollwege" entsprechen den Distanzen C < (A/2 - B/2) sowie F < (G/2 - H/2). Bei freier Befüllung der Ballonkörper kommt es in diesem Falle zu einer Berührung der Schulterflächen 6z und 8z, noch bevor die Neutrallage der Ballonkörper erreicht ist, die Schulterflächen, pressen sich dann, abhängig vom jeweiligen Fülldruck, einander an. Im optimalen Falle entspricht die montagerelevante Strecke J der Summe aus (A/2 - B/2) und (G/2 - H/2) - 5 bis -10 mm. Bei einer derartigen Verkürzung der Strecke J resultiert, unabhängig vom jeweiligen Kontraktionszustand des Pylorus, eine beidseitig in Richtung E federnde Auslenkung der Ballonkörper, sowie eine korrespondierend wirkende, anpressenden Rollbewegung D der Schulterflächen. Kommt es zu funktionellen Änderungen der Breite des pylorischen Schließmuskels, können diese durch die beschriebene Federung und Gegenrollung, bei weitgehend erhaltener ankernd und dichtend wirkender Spannung über dem Pylorus, kompensiert werden.

Der radiale Durchmesser des gastrischen Ballonkörpers 8 kann bevorzugt derart vergrößert sein, dass er den Bereich des Magen-Antrums, welcher sich dem Pylorus magenseitig anschließt, raumfordernd ausfüllt bzw. dessen Wandung unter eine moderate Spannung nimmt, wodurch dem Patienten ein anhaltendes Sättigungsgefühl vermittelt wird. Die dem Magen zugewandte Seite des Ballonkörpers 8 ist bevorzugt als trichterartig geformte Mündung T ausgeführt. Die Trichterform des den Nahrungsbrei aufnehmenden "Mundstückes" kann durch ein selbstaufrichtendes, stent-artiges Geflecht, welches im Mundstückbereich verbaut ist, in seiner Form und Wirkung stabilisiert werden.

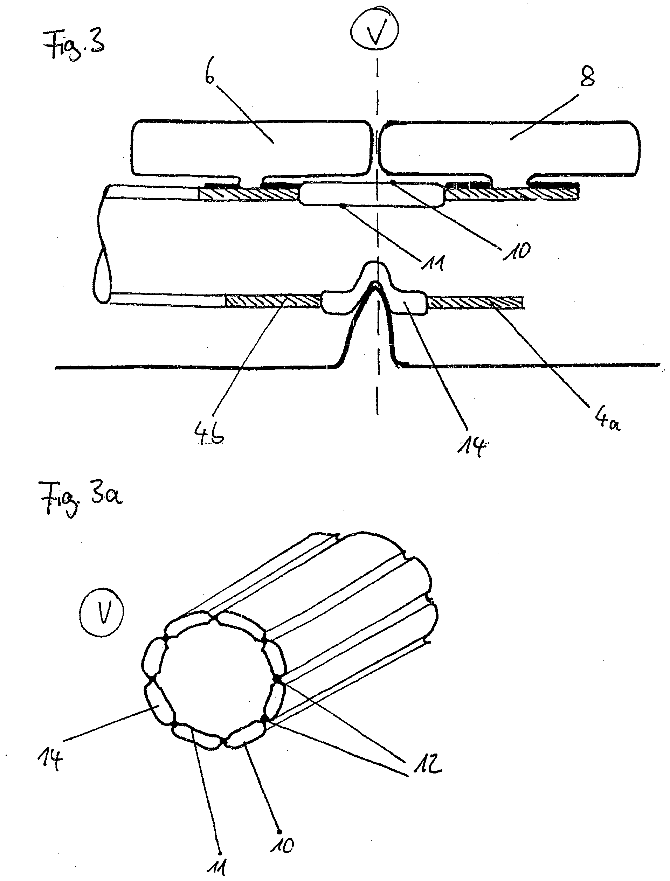

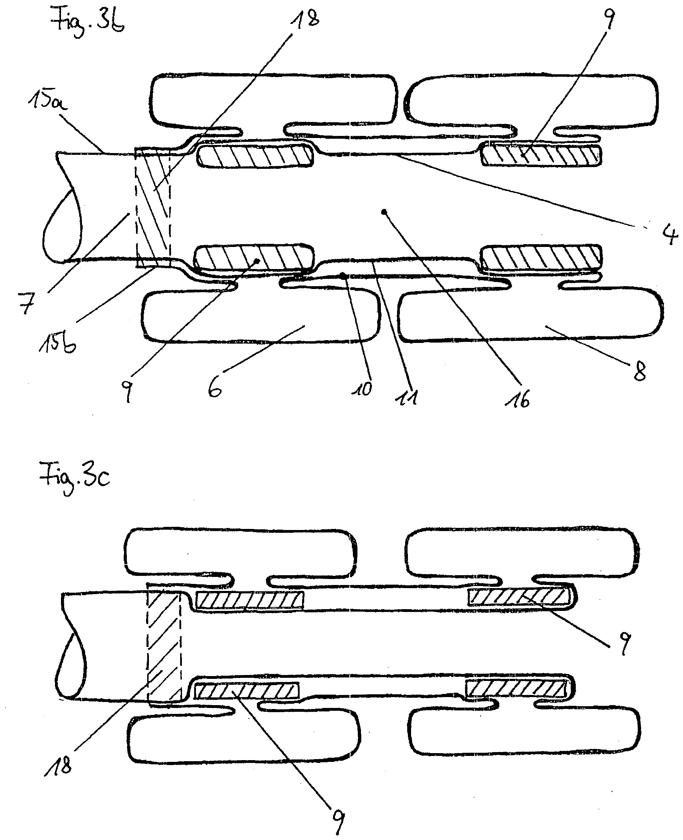

Fig. 3 zeigt eine Ausführungsform der Bypass-Vorrichtung, bei der der trans-pylorische Abschnitt des Durchleitungselementes 14 aus einer konzentrisch doppellagigen Anordnung von schlauchartigen Folien 10 und 11 besteht, die durch beispielsweise axial längsverlaufende Verschweißungslinien 12 oder auch durch gleichmäßig punktuell verteilte Verschweißungspunkte miteinander fest verbunden sind. Siehe hierzu die in Fig. 3a dargestellte Schnittebene V durch das Durchleitungselement 14. Die konzentrischen Schlauchfolien des Durchleitungselementes bilden so einen zylindrischen, befüllbaren Hohlschlauch 14 aus. Bei der Befüllung bzw. Beaufschlagung des in beschriebener Weise, luftmatratzenartig "abgesteppten" Hohlschlauches, richtet sich der quasi luftstabilisierte Schlauchkörper radial kreisrund auf. Neben der radialen Aufrichtung, kommt es zudem zu einer zudem zu einer axialen Entwindung über die Längsachse. Der Hohlschlauch 14 ist mit einem oder bevorzugt mit beiden Ballonsegmenten 6 und 8 volumenkommunizierend verbunden und wird über eine gemeinsame Füllvorrichtung befüllt. Von Vorteil für die besondere Gewebe- bzw. Organverträglichkeit ist, dass der lumenaufrichtende Effekt im trans-pylorischen Segment der Vorrichtung vom pylorischen Schließmuskel relativ leicht überwindbar ist, der Schließmuskel sich also relativ frei bewegen kann. Er wird, trotz einer fortwährenden, axialen Anpressung der Ballonsegmente 6 und 8 an die Schulterflächen des Pylorus, in seiner Fähigkeit zur Kontraktion kaum beeinträchtigt. Kommt es im Rahmen einer reflektorischen Magenentleerung zu einer Kontraktion des Magenantrums, wird diese vom magenseitigen Ballonelement aufgenommen und verursacht, für die Zeitdauer des Druckanstiegs, in den mit ihm verbundenen Kompartimenten, einen korrespondierenden Drucksteigerung. Der passagere Druckanstieg führt wiederum es zu einer Intensivierung der axialen Gegenrollung der Ballonsegmente, wodurch der die beiden Segmente verbindende Rohrschlauch 14 über seine Längsachse gestrafft und somit die Öffnung und Entwindung des Lumens unterstützt wird.

Die konzentrischen Schlauchfolien 10 und 11 bestehen bevorzugt aus PUR, beispielsweise im Härtebereich von Shore 80A bis 60D, bevorzugt im Bereich von 90 A bis 55D. Die Wandstärke der Folien sollte ca. 15 bis 50 µm, bevorzugt 20 bis 30 µm betragen. Als Materialtyp kann beispielsweise PUR der Familie Pellethane 2363 der Fa. Lubrizol Inc. verwendet werden.

Fig. 3b stellt eine weitere bevorzugte Ausführung des Vorrichtungstyps mit sich pneumatisch aufrichtendem, koaxial aufgebautem Rohrschlauch dar. Hier bestehen alle kompartimentbildenden Anteile der Vorrichtung, also sowohl die beiden Ballonsegmente 6 und 8, als auch das dazwischen liegende Durchleitungssegment, aus einer einzigen, als durchgängig ausgeformter Körper und anschließend in sich rückgestülpten Ballonfolie. Diese wird bereits bei der Herstellung mit allen funktionell und baulich erforderlichen Ausformungen versehen und auf ihr vollständiges Arbeitsmaß geformt. Das Ende 15a der ausgeformten Ballonfolie wird durch das gegenüber liegende Ballonkörperende 15b derart rückgestülpt, dass sich die verschiedenen Ballonsegmente in Konfiguration mit den Elementen des Durchleitungselementes in der abgebildeten Weise darstellen. Die zentralen Öffnungen der endständigen Ballonsegmente 6 und 8 werden jeweils durch elastisch aufrichtende Hülsenelemente 9 stabilisiert, welche in das durchleitende Lumen 16 der Kopfeinheit eingeschoben werden. Die Hülsenelemente dienen zur Aufnahme und Fixierung der endständigen Ballonsegmente, wobei bevorzugt die in den Figuren 1 und 2 beschriebenen Anweisungen gelten, und somit eine axiale, zum Pylorus hin gerichtete Rollbewegung der endständigen Ballonsegmente gewährleistet wird. Die beiden Folienlagen 10 und 11 gehen bei dieser Ausführung vollständig aus dem rückgestülpten Ballonkörper hervor. Das Ende 15a des ausgeformten Ballonkörpers kann zudem derart verlängert werden, dass daraus ohne erforderliche Fügestelle der trans-duodenale Bypass-Schlauch 7 hervorgeht.

Fig. 3c zeigt eine entsprechende Ausführung, bei der die lumenstabilisierenden Hülsenelemente 9 nicht im durchleitenden Lumen 16 angeordnet sind, sondern im Binnenraum des rückgestülpten Ballonkörpers verbaut werden.

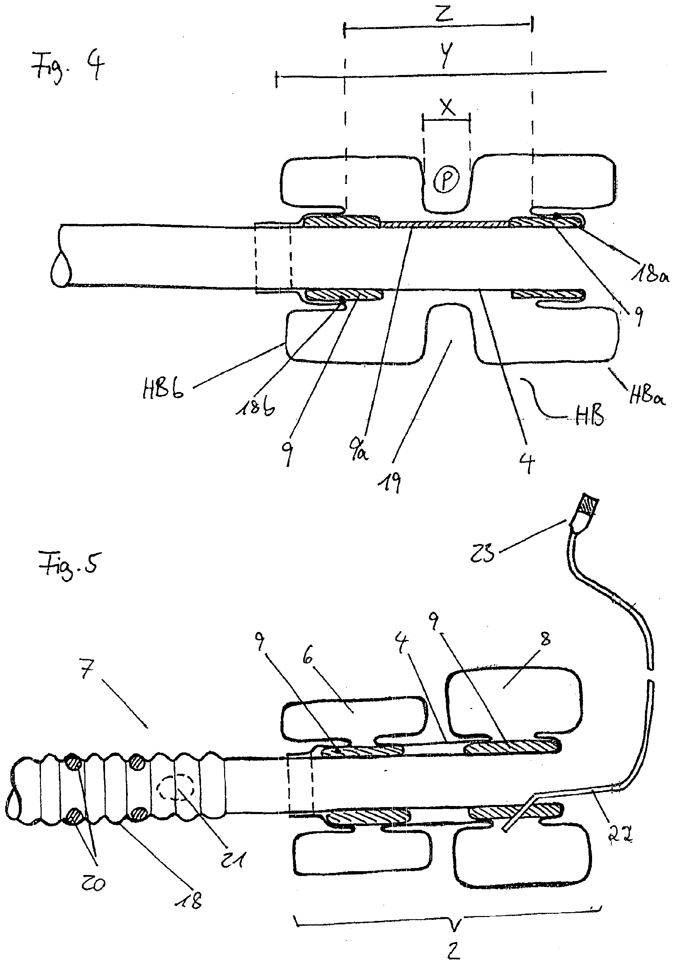

Fig. 4 zeigt eine weitere Ausführungsform der Vorrichtung, bei der alle Segmente der Kopfeinheit aus einem vollständig ausgeformten, in sich vollständig rückgestülpten, hantelförmigen Ballonkörper HB hervorgehen, wobei die endständigen Ballonsegmente HBa und HBb mit den Ballonenden 18a und 18b auf den tragenden Hülsenelementen 9 bzw. dem Durchleitungselement 4 aufgebracht sind. Über die Ballonenden 18a und 18b hinaus weist der Ballonkörper HB keine weitere Verbindung zum ballontragenden Durchleitungselement auf. Der mittige Abschnitt des Ballonkörpers wird bei der Formung mit einer Verjüngung 19 zur Aufnahme des pylorischen Schließmuskels versehen. Die Distanz X (Distanz zwischen den Schulterflächen der Verjüngung) soll (Y - Z)/2 nicht überschreiten. Die einander gegenüberstehenden Schulterflächen der Ballonsegmente HBa und HBb bewegen sich bei der Befüllung des Ballons in der dargestellten Ausführung auf einander zu und schmiegen sich dem in der Verjüngung sitzenden Schließmuskel radial und axial dichtend an. Bei zunehmendem Fülldruck des Ballons HB nimmt die Intensität der auf den Pylorus gerichteten Anpress- und Dichtungswirkung entsprechend zu.

Das Durchleitungselement 4 besteht bei der vorliegenden Ausführung aus einem durchgängig ausgeformten Rohrelement, welches sowohl die endständigen Elemente 9 zur Aufnahme der Enden des Ballonkörpers, als auch das zwischen ihnen liegende, dem Schließmuskel exponierte Element 9a ausbildet. Die zuvor beschriebene Verformbarkeit des Elementes 9a bei der Sphinkter-Kontraktion und die spontane elastische Aufrichtung nach Verformung wird bei dieser Ausführung der Vorrichtung berücksichtigt.

Fig. 5 zeigt den Aufbau einer erfindungsgemäßen Vorrichtung nach Fig. 3a in der Übersicht, bestehend aus den funktionellen Einheiten: trans-pylorische Fixiervorrichtung 2 (bestehend aus: gastrisches 8 und duodenales 6 Ballonelement mit tragenden Hülsenelementen 9, Durchleitungselement 4) sowie trans-duodenales Bypass-Element 7.

Das duodenale Bypass-Element 7 weist bevorzugt eine Wandungsstärke von 10 bis 80 µm, bevorzugt 15 bis 30 µm, besteht bevorzugt aus dem gleichen Material wie die mediumbeaufschlagten funktionellen Einheiten der Fixiervorrichtung, ist bevorzugt mit einem lumenaufrichtenden, ring- oder spiralförmig korrugierten Profil 18 versehen. Die Korrugation soll neben der radialen Aufrichtung des Lumens auch die spontane axiale Entwindung des Schlauches unterstützen. Die Länge des Schlauches ist bevorzugt derart bemessen, dass das aborale Ende bis in das terminale Duodenum oder auch in das beginnende Jejunum reicht. Zur Modifikation der Bypass-Wirkung kann das Element 7 auch mit Öffnungen 20 versehen sein, die den partiellen Übertritt von Nahrung in höhere Bereiche des Duodenums gestatten.

Die Bemaßung der trans-pylorischen Fixiervorrichtung wird bevorzugt wie folgt ausgeführt: Duodenales Ballonelement 6 (zylindrischer Durchmesser 25 bis 35 mm; zylindrische Länge 15 bis 50 mm, bevorzugt 20 bis 30 mm), gastrisches Ballonsegment 8 (zylindrischer Durchmesser 50 bis 80 mm, zylindrische Länge 30 bis 100 mm, bevorzugt 40 bis 60 mm), trans-pylorisches Segment 4 (Durchmesser 15 bis 30 mm, bevorzugt 20 bis 25 mm; Länge 5 bis 15 mm, bevorzugt 8 bis 12 mm).

Die befüllbaren bzw. beaufschlagbaren Kompartimente der Vorrichtung sind vorzugsweise miteinander kommunizierend verbunden. Die Befüllung erfolgt beispielsweise durch einen im Bereich des gastrischen Ballonsegmentes mündenden Befüllschlauch 22, der in seiner Länge so ausgelegt ist, dass er oral ausgeleitet und über seinen terminalen Verschluss 23 außerhalb des Körpers befüllt bzw. seine Befüllung nachreguliert werden kann.

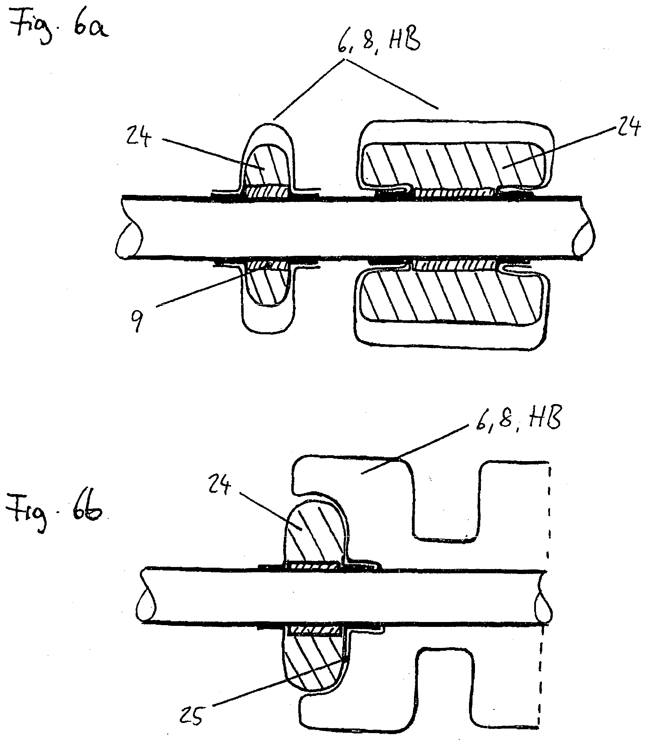

Fig. 6a und 6b beschreiben weitere Ausführungsformen der bisher beschriebenen Bautypen, bei denen insbesondere zur Verbesserung der trans-pylorischen Verankerung, innerhalb eines Ballonelementes oder eines ballonartig erweiterten Segmentes, wie beispielsweise 6, 8 oder auch HB, ein zusätzliches Ankerballonelement 24 angeordnet ist. Dieses Ballonelement wird durch eine separate Zuleitung befüllt. Hierzu kann ein Medium verwendet werden, welches sich vom Füllmedium des ihn umgebenden Ballons qualitativ unterscheidet. Das Ankerballonelement ist in bevorzugter Ausführung vollständig vom umgebenden Ballon umschlossenen. Das Ankerballonelement 24 kann optional auf beiden Seiten des Pylorus zur Anwendung kommen. Vorzugsweise besteht es aus Material, welches die Charakteristika einer Weichfolie aufweist, aber bei Befüllung eine vorgegebene Form bzw. Dimension nicht wesentlich überschreitet und somit eine unkontrollierte elastische Aufdehnung ausschließt. Steht als Funktionszweck die duodenalseitige Ankerung der Vorrichtung im Vordergrund, wird vorteilhaft eine diskoide Form gewählt. Ist zusätzlich eine zum Pylorus hin gerichtete Gegenrollung erwünscht, kann eine zylindrische Bauform zur Verwendung kommen, wobei die erfindungsgemäß beschriebenen, die Gegenrollung erzeugenden Montagevorschriften angewendet werden. Das Element 24 wird vorzugsweise mit einem nicht kompressiblen Medium, wie z.B. Wasser oder Öl befüllt, während das umgebende Ballonelement bevorzugt ein gasförmiges Medium enthält.

Alternativ zu einer vollständigen Einhausung des Ballons 24 in einen umgebenden Ballon, kann der Ballon 24 auch nur anteilig, wie in Fig. 6b gezeigt, vom umgebenden Ballon umschlossen sein. Die Kontaktfläche 25 zwischen beiden Ballonhüllen kann fix verbunden oder auch unverbunden sein. In einer weniger bevorzugten Kombination eines primär ankernd wirkenden Ballonelementes 24 mit einem primär dichten wirkenden Ballonelement 6,8 oder HB, ist ebenfalls eine sequentielle Anordnung der Ballonelemente auf dem Schaftelement denkbar.

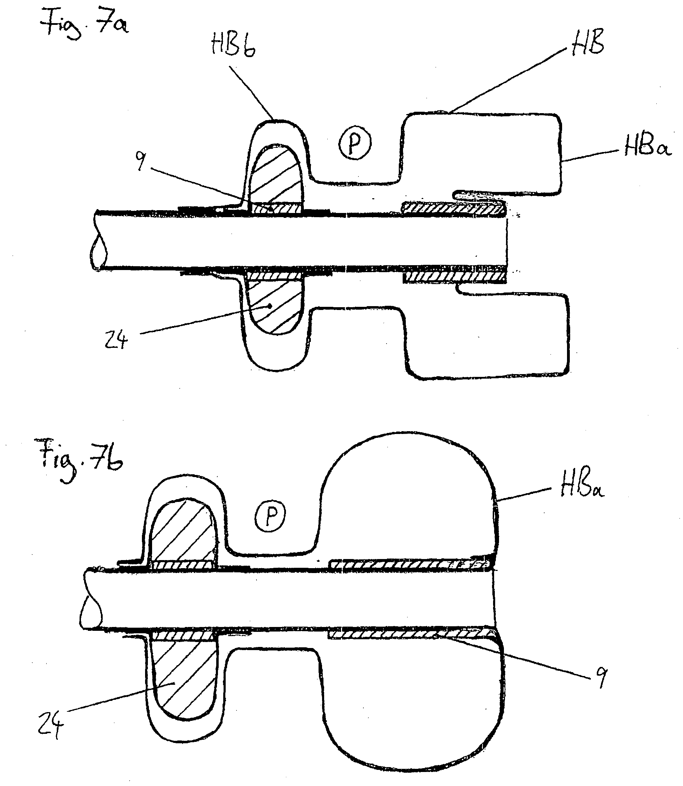

Abb. 7a und 7b zeigen eine bevorzugte Ausführungsform der Vorrichtung in Anlehnung an die in Fig. 4 und Fig. 6 beschriebenen Bautypen, bei der das Durchleitungselement 4 eine hantelförmigen Ballon HB trägt, welcher derart auf dem Durchleitungselement montiert wird, dass sich die in Fig. 4 beschriebene, zum Pylorus P hin gerichtete Gegenrollung der endständigen Ballonsegmente HBa und HBb einstellt. Das zusätzliche Ballonelement 24 wird in der vorgestellten Ausführung nur duodenalseitig eingebaut und hat eine diskoide Form. Die Kontur des Ballons 24 entspricht bevorzugt der Kontur des den Ballon umhüllenden, duodenalseitigen Ballonsegmentes HBb. Der Ballon HBb sollte die Durchmessermaße des inneren Ballons 24 überschreiten, während seine axiale Länge der des Ballons 24 in bevorzugter Ausführung entspricht. Der Ballon 24 wird unmittelbar hinter dem Pylorus im Bulbus duodeni platziert. Die Gegenrollung des magenseitigen Ballonsegmentes HBa zum Pylorus hin, wird analog zu der in Fig. 4 beschriebenen Bauweise, durch eine Inversion des Ballonendes des Ballonsegmentes HBa erreicht. Bei der trans-pylorischen Applikation der beschriebenen Ausführungsform wird initial das Element 24 mit Flüssigkeit befüllt und die Vorrichtung so duodenalseitig in ihrer Position gesichert. Anschließend wird der umgebende Ballon mit einer Luftfüllung beaufschlagt, wodurch sich dieser sowohl radial dichtend an die exponierte Schleimhaut schmiegt, als sich auch axial an die Schulterflächen des Pylorus presst. Der umgebende Ballon übernimmt so, neben der Wirkung als gastrisches Widerlager bzw. als raumfordernder Körper im Magen-Antrum, eine überwiegend dichtende Funktion. Seine Wandstärke liegt vorzugsweise im Bereich von 10 bis 30 µm, weniger bevorzugt im Bereich von 30 bis 100µm. Die Materialhärte sollte im Bereich von Shore 80A bis 75D liegen, bevorzugt aber 85A bis 65D aufweisen. Die Materialhärte des Ankerballons 24 liegt vorzugsweise im Bereich 95A bis 65D. Die besondere Kombination von Dünnwandigkeit und Materialhärte des umgebenden hantelförmigen Ballons HB erlaubt es magenseitig herrschende Kräfte großflächig aufzunehmen und im Bereich des pylorischen Durchgangs sowie im duodenalseitigen Abschnitt des Ballonkörpers zur effizienten Dichtung gegen Mageninhalt zu nutzen. Kommt es zu einer Kontraktion des Magen-Antrums, wird die Kontraktionskraft magenseitig aufgenommen und Füllvolumen, unter einer entsprechenden Steigerung des Fülldrucks, in die korrespondierenden Ballonanteile verdrängt. Die beschriebene Materialhärte begrenzt dabei die Aufdehnung der Ballonhülle und beugt somit der partiellen Entleerung eines Ballonanteils in einen angrenzenden Ballonanteil vor. Die besondere Dünnwandigkeit der Ballonhülle HB erlaubt es, sämtliche Segmente des Ballonkörpers residual zu dimensionieren, was einer Ausformung der jeweiligen Segmente über das erforderliche bzw. anzunehmende anatomische Maß hinaus entspricht. Der residual ausgeformte Ballonkörper liegt den Schleimhautflächen dann unter Einfaltung der Ballonhülle an, wobei dennoch ein dichtender Verschluss gewährleistet wird. Bei entsprechender Volumenverschiebung aus dem magenseitigen in den pylorischen und duodenalseitigen Anteil, kann so, durch das residuale Übermaß ermöglicht, eine kraftaufnehmende elastische Aufdehnung der Hülle vermieden werden, und die jeweilig magenseitig wirkende Kraft im vollen Umfang zur pylorisch und duodenalen Dichtung eingesetzt werden. Bei der Gestaltung des Durchleitungselementes 4 bzw. der Hülsenelemente 9, muss deren elastische Selbstaufrichtungswirkung so konstruiert werden, dass bei einer passageren, kontraktionsbedingten Drucksteigerung innerhalb des Ballons HB ein Kollaps des durchleitenden Lumens 16 vermieden werden kann.

Fig. 7b zeigt eine analoge Ausführungsform, bei der auf die Gegenrollung des magenseitigen Ballonsegmentes HBa nahezu vollständig oder vollständig verzichtet wird und lediglich eine radial wirkende Dichtung der umschließenden Ballonhülle zum Pylorus wirkt. Die in Fig. 7a beschriebene Inversion der magenseitigen Schaftenden der Ballonhülle HB kommt hier nicht zur Anwendung.

Fig. 8 zeigt eine an Fig. 7b angelehnte Ausführungsvariante, bei der ein ankerndes Ballonelement 24 nicht nur duodenalseitig, sondern auch magenseitig platziert wird. Das magenseitige Element 24a weist in der bevorzugten Ausführung ebenfalls eine diskoide Form auf und kann in radialen Bemaßung den Raumverhältnissen des Magenantrums angepasst sein. Beide Elemente 24 und 24a können separat befüllbar sein oder alternativ über eine gemeinsame Zuleitung mit einem Medium beaufschlagt werden. Ferner können beide Elemente durch eine hantelförmige Verjüngung 24b, welche im pylorischen Durchgang platziert wird, miteinander verbunden sein. Für die Ausführung der Elemente 24 und 24a wird bevorzugt ebenfalls ein Polyurethan geringer Compliance verwendet, um zu vermeiden, dass sich bei Belastung eines Ballonkompartiments Volumen in kommunizierende Kompartimente verschiebt und die es dort zu einer unerwünschten elastischen Dehnung der Ballonhülle kommt. Die dargestellte Variante mit beidseitiger Flankierung des Pylorus mit einem ankernden Ballonelement 24 ermöglicht eine zusätzliche Sicherungsfunktion, falls die die Ballonelemente 24 einhausende Ballonhülle HB im Schaden nimmt und ihre Füllung verliert. Durch die bilaterale Anordnung der Elemente 24 bleibt die trans-pylorische Platzierung der Vorrichtung gewährleistet.

Fig. 9 stellt eine beispielhafte Ausführungsform eines für die endoskopische Applikation erforderlichen Abwurfmechanismus 26 dar, der als zylindrischer Kopplungsballon 27 ausgeführt ist und auf den Schaft 28 eines Endoskopes aufgezogen wird. Auf dem distalen Ende des Endoskopes positioniert, presst er sich bei Befüllung der Kanalwandung des durchleitenden Segmentes 4 der Bypass-Kopfeinheit an und stellt so die Kopplung zwischen Bypass und Endoskop her. Wird die Befüllung aus dem Ballon 27 entnommen, löst sich sie Kopplung und der Endoskopschaft kann aus dem Kanal der Kopfeinheit zurückgezogen oder auch duodenalwärts vorgeschoben werden. Der Ballon wird durch eine Zuleitung 29 von außerhalb des Körpers befüllt.

Um eine sichere Positionierung des Bypass auf dem Abwurfmechanismus zu gewährleisten, kann der Kopplungsballon 27 mit einer proximalen, schulterartigen Ausformung 30 versehen sein, die als mechanisch wirksamer Anschlag dient. Eine entsprechende Anschtagfunktion kann auch unabhängig von einem durchgehend zylindrisch ausgeführten Kopplungsballon, als sich separat entfaltender Widerlagerballon in das proximale Ende der Kopplungseinheit integriert sein. Eine entsprechende Ausformung oder ein entsprechender separater Widerlagerballon kann ergänzend auch distal des Bypass ausgeformt bzw. angeordnet sein. Der Kopplungsballon 27 nimmt somit eine Hantelform an, die die Bypass-Vorrichtung im verjüngten Bereich tragend aufnimmt. Die Hülle des Kopplungsballons ist vorzugsweise aus einem PUR-basierten Material niedriger Compliance ausgeformt, und wird bevorzugt mit einem flüssigen Medium beaufschlagt.

Im Folgenden wird beispielhaft die endoskopische Platzierung einer Bypass-Vorrichtung nach Abbildung 7a oder 7b beschrieben.

Hat die der Endoskopspitze aufsitzende Bypass-Vorrichtung den Magenbinnenraum erreicht, wird zuerst der äußere hantelförmige Ballon HB mit vorzugsweise 60 bis 80 % seines frei entfalteten Volumens befüllt. Der so spannungslos mit Luft befüllte Ballon HB wird nun mit dem Endoskop soweit in den Pylorus eingeführt, bis die pylorusseitige Schulter des gastrischen Ballonsegmentes HBa an den Pylorus anschlägt und ein weiteres endoskopisches Vorschieben des Bypass verhindert. Der sich beim Anschlag der Ballonschulter im Magenausgangsbereich einstellende Widerstand wird vom Anwender wahrgenommen und bestätigt die korrekte trans-pylorische Platzierung der Vorrichtung. Es erfolgt dann die Befüllung des innliegenden Ankerballons 24 mit einem flüssigen Medium. Anschließend wird der äußere Ballon HB bis auf sein endgültiges Arbeitsmaß gefüllt.

Ist die Kopfeinheit der Bypass-Vorrichtung so in ihrer trans-pylorischen Position gesichert, wird der Bypass durch Entleerung des Kopplungsballons 27 vom Applikator- oder Endoskopschaft freigegeben und die Applikator- oder Endoskopspitze weiter in das Duodenum eingeführt. Hierbei kann der duodenal durchleitende Anteil 7 der Bypass-Vorrichtung mit einem entsprechenden Instrument an der Applikator- oder Endoskopspitze gegriffen und in das Duodenum hinein transportiert werden. Die Vorrichtung sieht hierzu einen geeigneten Fortsatz am unteren freien Ende des trans-duodenalen Schlauches 7 vor. Ist die jeweils mögliche duodenale Einführtiefe des Applikator- oder Endoskopschaftes erreicht, kann das freie untere Schlauchende des Bypasses durch Vorschub des Greifinstrumentes weiter in den Zwölffingerdarm hinein befördert und dort schließlich abgeworfen werden.

Ist der transduodenal durchleitende Anteil 7 der Vorrichtung so teilweise oder vollständig entfaltet, kann zur weiteren lumen-öffnenden Entfaltung des Anteils 7 eine Luftinsufflation in den Schlauch oder auch eine Spülung erfolgen. Dies erfolgt bevorzugt derart, dass der auf dem Applikator- oder Endoskopschaft aufsitzende koppelnde Ballon 27 im Lumen der Kopfeinheit platziert und dort für die Dauer der Insufflation bzw. Spülung dichtend geblockt wird. Somit kann die lumenaufrichtende und -ausrichtende Befüllung des duodenalseitigen Schlauchanteils 7 ohne jeden Reflux in den Magen erfolgen.

**Bezugszeichenliste**

| | | | |
|---|---|---|---|
| 1 | Bypass-Vorrichtung | 19 | Verjüngung |
| 2 | Fixiereinheit | 20 | Öffnung |
| 4 | Durchleitungselement | 22 | Befüllschlauch |
| 4a | magenseitiges Ende | 23 | terminaler Verschluss |
| 4b | duodenalseitiges Ende | 24 | Ankerballonelement |
| 4kk | Korrugation | 24a | magenseitiges Element |
| 5 | Widerlagerelement | 24b | Verjüngung |
| 5z | magenseitige Schulter | 25 | Kontaktfläche |
| 6 | duodenales Ballonelement | 26 | Abwurfmechanismus |
| 6x | Abschlusskante | 27 | Kopplungsballon |
| 6y | Abschlusskante | 28 | Schaft |
| 6z | pylorusseitige Schulter | 29 | Zuleitung |
| 7 | Schlauchelement | A | zylindrische Kontaktfläche |
| 8 | gastrisches Ballonelement | B | Distanz |
| 8z | Schulter | C | Strecke |
| 9 | Element, Hülsenelement | D | Rollbewegung, Pfeil |
| 10 | schlauchartige Folie | E | Gegenrichtung, Pfeil |
| 11 | schlauchartige Folie | HB | hantelförmiger Ballonkörper |
| 12 | Verschweißungslinie | HBa | Ballonsegment |
| 14 | Durchleitungselement | HBb | Ballonsegment |
| 15a | Ende | J | Strecke |
| 15b | Ende | M | Magenseite |
| 16 | Lumen | P | Pylorus |
| 18 | korrugiertes Profil | T | Mündung |
| 18a | Ballonende | X | Distanz |
| 18b | Ballonende | Z | Duodenalseite |

## Patentansprüche

1. Bypass-Vorrichtung (1) zur Aufnahme von Nahrungsbrei aus dem Magen oder Gaster und zur bypass-artigen Fortleitung des Nahrungsbreis durch den Magenpförtner oder Pylorus in oder durch den Zwölffingerdarm oder das Duodenum eines Patienten, umfassend ein schlauchförmiges, radial kollabierbares und sich selbst aufrichtendes, den Pylorus durchsetzendes, trans-pylorisches Durchleitungselement (4,7) mit einem zentralen Durchleitungslumen für den Nahrungsbrei, sowie Fixierungselemente zur Verankerung des trans-pylorischen Durchleitungselements (4,7) an dem Pylorus, bestehend aus einem magenseitig oder proximal des Pylorus angeordneten, ringförmigen, gastrischen Ankerelement zur Verankerung des trans-pylorischen Durchleitungselements (4,7) proximal des Pylorus, mit einem gastrischen Ballonsegment (8), welches einen mit einem Medium befüllbaren Hohlraum von ringförmiger Struktur bereichsweise umgrenzt, sowie aus einem darmseitig oder distal des Pylorus im Zwölffingerdarm liegenden, ringförmigen, duodenalen Ankerelement zur Verankerung des trans-pylorischen Durchleitungselements (4,7) distal des Pylorus, mit einem duodenalen Ballonsegment (6), welches einen mit einem Medium befüllbaren Hohlraum von ringförmiger Struktur bereichsweise umgrenzt, **dadurch gekennzeichnet, dass** das gastrische Ballonsegment (8) und/oder das duodenale Ballonsegment (6) das trans-pylorische Durchleitungselement (4,7) radial außerhalb umgibt, entlang einer in toroidaler Richtung umlaufenden Linie nicht geschlossen ist, und derart mit dem Durchleitungselement (4,7) verbunden ist, dass letzteres einen Teil der Umfassung des betreffenden, toroidalen Hohlraums bildet, wobei das Durchleitungselement (4,7) zumindest in seinem den gastrischen und/oder duodenalen Hohlraum umfassenden Bereich derart ausgebildet oder verstärkt ist, dass es dort eine höhere Strukturstabilität oder Selbstaufrichtungsfähigkeit aufweist als das betreffende Ballonsegment (6,8) in der unmittelbaren Umgebung, und wobei wenigstens ein Ballonsegment (6,8) (i) wenigstens einen rundum laufenden, ringförmig geschlossenen, an dem trans-pylorischen Durchleitungselement (4,7) anliegenden, freien Kantenbereich aufweist, welcher derart umgestülpt ist, dass er mit seiner dem betreffenden Hohlraum zugewandten Innenseite an dem trans-pylorischen Durchleitungselement (4,7) flächig anliegt, und (ii) mit dem trans-pylorischen Durchleitungselement (4,7) dichtend verbunden ist.

2. Bypass-Vorrichtung (1) nach einem der vorhergehenden Ansprüche, **dadurch gekennzeichnet, dass** zwei freie Stirnkanten wenigstens eines Ballonsegmentes (6,8) jeweils nach innen in das betreffende Ballonsegment (6,8) hinein umgestülpt sind, wobei vorzugsweise zwischen einer freien Stirnkante und deren Umstülpung in das betreffende Ballonsegment (6,8) hinein eine zweite, entgegengesetzte Umstülpung liegt, also in Richtung aus dem betreffenden Ballonsegment (6,8) heraus.

3. Bypass-Vorrichtung (1) nach einem der vorhergehenden Ansprüche, **dadurch gekennzeichnet, dass** ein Ballonsegment (6,8) derart präformiert ist,
a) dass es in bestimmten ringförmigen Abschnitten unterschiedliche Umfangslängen aufweist, insbesondere dass es im Bereich zweier freier Stirnkanten jeweils eine kleiner Umfangslänge aufweist als in einem dazwischen liegenden Bereich des Ballonmantels, der einen sich nach außen stülpenden Abschnitt des Ballonsegments (6,8) bildet, und/oder derart
b) dass es in bestimmten ringförmigen Abschnitten unterschiedliche Stärken aufweist, insbesondere dass es im Bereich zweier freier Stirnkanten jeweils eine größere Stärke aufweist als in einem dazwischen liegenden Bereich des Ballonmantels, der einen sich nach außen stülpenden Abschnitt des Ballonsegments (6,8) bildet, und/oder derart,
c) dass bei bis zu seinem vorgeformten Volumen expandiertem Hohlraum der Querschnitt durch diesen Hohlraum eine größere Erstreckung in axialer Richtung aufweist als in radialer Richtung, bezogen auf die Längsachse des Durchleitungselements (4,7).

4. Bypass-Vorrichtung (1) nach einem der vorhergehenden Ansprüche, **dadurch gekennzeichnet, dass** ein oder beide Ballonsegmente (6,8) derart ausgebildet und/oder präformiert sowie in derartiger Weise auf dem Durchleitungselement (4,7) fixiert ist (sind), dass sie eine zum Pylorus hin rollende Affinität haben, insbesondere eine aufeinander zu rollende Tendenz.

5. Bypass-Vorrichtung (1) nach einem der vorhergehenden Ansprüche, **dadurch gekennzeichnet, dass** innerhalb wenigstens eines, von einem Ballonsegment (6,8) bereichsweise umgrenzten Hohlraums ein zusätzliches, inneres Kissen oder Ballonelement angeordnet ist, welches mit einem anderen Füllmedium befüllt oder befüllbar ist als der jenes aufnehmende Hohlraum selbst, vorzugsweise wobei das innere Kissen oder Ballonelement auf dem trans-pylorischen Durchleitungselement (5,7) rundum dichtend fixiert ist, und/oder ein kleineres Volumen umschließt und/oder mit einem kleineren Volumen präformiert ist als das von dem jenes aufnehmenden Hohlraum abgestützte, außenliegende Ballonsegment (6,8).

6. Bypass-Vorrichtung (1) nach Anspruch 5, **dadurch gekennzeichnet, dass** das innere Ballonelement
- mit einem flüssigen Medium befüllbar ist, und/oder
- torusförmig ausgebildet ist, und/oder
- im Bereich einer oder beider seiner ringförmig umlaufenden Stirnkanten mit seiner Innenseite, welche seinem Füllmedium zugewandt ist, auf dem trans-pylorischen Durchleitungselement (5,7) flächig anliegt und dort fixiert ist.

7. Bypass-Vorrichtung (1) nach einem der vorhergehenden Ansprüche, **dadurch gekennzeichnet, dass** das gastrische Ballonsegment (8) und das duodenale Ballonsegment (6) zu einem einzigen Ballon vereinigt sind, der hantelförmig präformiert ist, mit einer etwa mittigen, rundum laufenden Verjüngung zur Aufnahme des Pylorus-Schließmuskels.

8. Bypass-Vorrichtung (1) nach einem der vorhergehenden Ansprüche, **gekennzeichnet durch** wenigstens eine Zuleitung oder einen Befüllschlauch (22) zu wenigstens einem toroidalen Hohlraum, so dass eine oder beide Ballonsegmente (6,8) nach der Platzierung der trans-pylorischen Bypass-Vorrichtung (1), insbesondere des trans-pylonischen Durchleitungselements (4,7) und/oder der Fixiereinheit (2), befüllbar sind, vorzugsweise wobei das orale oder proximale Ende (23) des Befüllschlauches (22) in den Magen einführbar und/oder dort abwerfbar ist, und/oder vorzugsweise wobei wenigstens eine Zuleitung zu wenigstens einem toroidalen Hohlraum, insbesondere ein Befüllschlauch (22), mit einem Rückschlagventil versehen ist.

9. Bypass-Vorrichtung (1) nach einem der vorhergehenden Ansprüche, **dadurch gekennzeichnet, dass** das trans-pylorische Durchleitungselement (4,7) das gastrische Ankerelement mit dem duodenalen Ankerelement verbindet.

10. Bypass-Vorrichtung (1) nach einem der vorhergehenden Ansprüche, **dadurch gekennzeichnet, dass** die Wandstärke des trans-pylorischen Durchleitungselements (4,7) dicker ist als die Wandstärke des gastrischen Ballonsegments (8) und/oder als die Wandstärke des duodenalen Ballonsegments (6), beispielsweise wenigstens doppelt so dick, vorzugsweise wenigstens 5 mal so dick, insbesondere wenigstens 10 mal so dick.

11. Bypass-Vorrichtung (1) nach einem der vorhergehenden Ansprüche, **dadurch gekennzeichnet, dass** das trans-pylorische Durchleitungselement (4,7)
a) schlauchförmig präformiert ist, so dass es bei Befüllung eines oder beider Ballonsegmente unter dem dortigen Fülldruck nicht kollabiert, und/oder
b) gewellt oder korrugiert ist, so dass es selbstaufrichtende Eigenschaften hat, aber in faltenbalgartiger Weise zusammenschiebbar oder raffbar ist, und/oder
c) im Bereich eines oder beider Ballonsegmente durch eine Hülse oder eine ring- oder spiralförmige Feder versteift ist, so dass es bei Befüllung eines oder beider Ballonsegmente unter dem dortigen Fülldruck nicht kollabiert.

12. Bypass-Vorrichtung (1) nach einem der vorhergehenden Ansprüche, **dadurch gekennzeichnet, dass** das trans-pylorische Durchleitungselement (4,7) eine koaxial doppellagige Schlauchfolienanordnung aufweist, vorzugsweise wobei die beiden Schlauchfolienlagen des trans-pylorischen Durchleitungselements (4,7) zumindest bereichsweise miteinander verbunden sind, vorzugsweise durch punktuelle, linienartige oder flächige Verbindungen, insbesondere in dem den Pylorus durchsetzenden Abschnitt, und/oder vorzugsweise wobei die Schlauchfolienlagen des trans-pylorischen Durchleitungselements (4,7) zusammen mit einem oder beiden Ballonsegmenten aus einem gemeinsamen Folienschlauch geformt sind.

13. Bypass-Vorrichtung (1) nach einem der vorhergehenden Ansprüche, **dadurch gekennzeichnet, dass** das trans-pylorische Durchleitungselement (4,7) und/oder das gastrische Ankerelement oder Ballonsegment (8) und/oder das duodenale Ankerelement oder Ballonsegment (6) aus Polyurethan bestehen.

14. Bypass-Vorrichtung (1) nach einem der vorhergehenden Ansprüche, **gekennzeichnet durch** wenigstens eine an dem trans-pylorischen Durchleitungselement (4,7) und/oder in oder an der Fixiereinheit (2) proximal und/oder distal des Pylorus platzierte, röntgendichte Markierung, um die korrekte trans-pylorische Lage der trans-pylorischen Bypass-Vorrichtung (1), insbesondere des trans-pylonischen Durchleitungselements (4,7) und/oder der Fixiereinheit (2), anzuzeigen.

15. Bypass-Vorrichtung (1) nach einem der vorhergehenden Ansprüche als Bestandteil eines Systems mit einem Applikator zur trans-pylorischen Platzierung der trans-duodenalen Bypass-Vorrichtung (1) nach einem der vorangehenden Ansprüche im Bereich des Pylorus eines Patienten, **gekennzeichnet durch** ein Applikator-Element in Form eines Endoskops oder Katheters, mit einem langgestreckten Schaft, auf dessen Außenfläche die Bypass-Vorrichtung (1) derart aufsetzbar und/oder aufsteckbar ist, dass der Applikatorschaft das zentrale Durchleitungslumen der Bypass-Vorrichtung (1) ganz oder teilweise durchsetzt.

16. Bypass-Vorrichtung (1) nach Anspruch 15, **dadurch gekennzeichnet, dass** die Bypass-Vorrichtung
a) auf dem Applikatorschaft reversibel, d.h. lösbar, gehalten ist, und/oder
b) auf dem Applikatorschaft klemmend gehalten ist, wobei zur klemmenden Fixierung der Bypass-Vorrichtung (1) an der Außenseite des Applikatorschaftes ein ringförmiger, von extrakorporal befüllbarer, spaltüberbrückender Ballon vorgesehen ist, welcher sich bei aufgesetzter oder aufgesteckter Bypass-Vorrichtung (1) in dem ringförmigen Spalt zwischen dem Applikatorschaft einerseits und dem trans-pylorischen Durchleitungselements (4,7) andererseits befindet, insbesondere radial innerhalb der Fixiereinheit (2) und/oder radial innerhalb des vorderen Endes des trans-duodenal durchleitenden Schlauches (7).

17. Bypass-Vorrichtung (1) nach einem der Ansprüche 15 oder 16, **dadurch gekennzeichnet, dass** der Applikator aufweist:
a) ein Ventil zum Entlüften des die Bypass-Vorrichtung (1) auf dem Applikatorschaft klemmenden Ballons, und/oder
b) Spülöffnungen, vorzugsweise distal des spaltüberbrückenden Ballons, durch welche über eine längs des Applikators verlaufende Spülleitung von extrakorporal Flüssigkeit in das Duodenum einspülbar ist, um den distalen Abschnitt des Durchleitungselements (4,7) zu entfalten, und/oder
c) wenigstens eine röntgendichte Markierung im Bereich des distalen Endes, so dass beim Vorschub des Applikatorschaftes dessen Lage überprüfbar ist.

## Claims

1. Bypass device (1) for accepting chyme from the stomach and for bypass-type conducting of the chyme through the pylorus in or through the duodenum of a patient, comprising a tubular, radially collapsible and self-erecting transpyloric conducting element (4, 7), which penetrates the pylorus, having a central conducting lumen for the chyme, and fixing elements for anchoring the transpyloric conducting element (4, 7) on the pylorus, consisting of an annular gastric anchor element, which is arranged on the gastric side or proximal to the pylorus, for anchoring the transpyloric conducting element (4, 7) proximally to the pylorus, having a gastric balloon segment (8), which regionally delimits a cavity of annular structure fillable with a medium, and also an annular duodenal anchor element, which is located on the intestine side or distally to the pylorus in the duodenum, for anchoring the transpyloric conducting element (4, 7) distally to the pylorus, having a duodenal balloon segment (6), which regionally delimits a cavity of annular structure fillable with a medium, **characterized in that** the gastric balloon segment (8) and/or the duodenal balloon segment (6) encloses the radial exterior of the transpyloric conducting segment (4, 7), is not closed along a circumferential line in the toroidal direction, and is connected to the conducting element (4, 7) such that the latter forms a part of the enclosure of the relevant toroidal cavity, wherein the conducting element (4, 7) is formed or reinforced at least in its region encompassing the gastric and/or duodenal cavity such that it has a higher structural stability or self-erection capability there than the relevant balloon segment (6, 8) in the immediate surroundings, and wherein at least one balloon segment (6, 8) has a free edge area extending circumferentially, which is closed in a ring shape and presses against the transpyloric conducting element (4, 7), which free edge area (i) is everted such that it presses flatly with its inner side, which faces the regarding cavity, against the transpyloric conducting segment (4, 7), and (ii) is connected to the transpyloric conducting element (4, 7) to form a seal.

2. Bypass device (1) according to any one of the preceding claims, **characterized in that** two free end edges of at least one balloon segment (6, 8) are each everted inward into the relevant balloon segment (6, 8), wherein preferably a second, opposing eversion is located between a free end edge and the eversion thereof into the relevant balloon segment (6, 9), i.e., in the direction out of the relevant balloon segment (6, 8).

3. Bypass device (1) according to any one of the preceding claims, **characterized in that** a balloon segment (6, 8) is preformed such
a) that it has different circumferential lengths in certain annular sections, in particular it has a smaller circumferential length in the region of two free end edges than in the region of the balloon jacket located in between, which forms an outwardly everted section of the balloon segment (6, 8), and/or such
b) that it has different thicknesses in certain annular sections, in particular it has a greater thickness in the region of two free end edges than in an interposed region of the balloon jacket, which forms an outwardly everted section of the balloon segment (6, 8), and/or such
c) that if its cavity is expanded up to its preformed volume, the cross section through this cavity has a greater extension in the axial direction than in the radial direction, in relation to the longitudinal axis of the conducting element (4, 7).

4. Bypass device (1) according to any one of the preceding claims, **characterized in that** one or both balloon segments (6, 8) are formed and/or preformed and are fixed to the conducting element (4, 7) in such a way that they have an affinity to roll toward the pylorus, in particular a tendency to roll toward one another.

5. Bypass device (1) according to any one of the preceding claims, **characterized in that**, within at least one cavity regionally delimited by a balloon segment (6, 8), an additional, inner cushion or balloon element is arranged, which is filled or fillable with a different filling medium than that of the cavity accepting it, preferably wherein the inner cushion or balloon element is peripherally fixed to form a seal on the transpyloric conducting element (5, 7), and/or encloses a smaller volume and/or is preformed having a smaller volume than the external balloon segment (6, 8) supported by the accepting cavity.

6. Bypass device (1) according to claim 5, **characterized in that** the inner balloon element
- is fillable with a liquid medium, and/or
- is formed as toroidal, and/or
- in the region of one or both of its annular circumferential end edges, presses flatly with its inner side, which faces toward its filling medium, on the transpyloric conducting element (5, 7) and is fixed there.

7. Bypass device (1) according to any one of the preceding claims, **characterized in that** the gastric balloon segment (8) and the duodenal balloon segment (6) are united to form a single balloon, which is preformed in a dumbbell shape, having an approximately central, circumferentially extending constriction to accept the pylorus sphincter.

8. Bypass device (1) according to any one of the preceding claims, **characterized by** at least one supply line or a filling tube (22) to at least one toroidal cavity, so that one or both balloon segments (6, 8) are fillable after the placement of the transpyloric bypass device (1), in particular of the transpyloric conducting element (4, 7) and/or the fixing unit (2), preferably wherein the oral or proximal end (23) of the filling tube (22) can be inserted into the stomach and/or dropped therein, and/or preferably wherein at least one supply line to at least one toroidal cavity, in particular a filling tube (22), is provided with a check valve.

9. Bypass device (1) according to any one of the preceding claims, **characterized in that** the transpyloric conducting element (4, 7) connects the gastric anchor element to the duodenal anchor element.

10. Bypass device (1) according to any one of the preceding claims, **characterized in that** the wall thickness of the transpyloric conducting element (4, 7) is thicker than the wall thickness of the gastric balloon segment (8) and/or than the wall thickness of the duodenal balloon segment (6), for example, at least twice as thick, preferably at least 5 times as thick, in particular at least 10 times as thick.

11. Bypass device (1) according to any one of the preceding claims, **characterized in that** the transpyloric conducting element (4, 7)
a) is preformed in a tube shape, so that it does not collapse under the filling pressure therein upon filling of one or both balloon segments, and/or
b) is wavy or corrugated, so that it has self-erecting properties, but can be collapsed or gathered in the manner of a folded bellows, and/or
c) is stiffened in the region of one or both balloon segments by a sleeve or an annular or spiral spring, so that it does not collapse under the filling pressure therein upon filling of one or both balloon segments.

12. Bypass device (1) according to any one of the preceding claims, **characterized in that** the transpyloric conducting element (4, 7) has a coaxial double-layered tube film arrangement, preferably wherein the two tube film layers of the transpyloric conducting element (4, 7) are at least regionally connected to one another, preferably by punctiform, linear, or planar connections, in particular in the section penetrating the pylorus, and/or preferably wherein the tube film layers of the transpyloric conducting segment (4, 7) together with one or both balloon segments, are formed from a common film tube.

13. Bypass device (1) according to any one of the preceding claims, **characterized in that** the transpyloric conducting segment (4, 7) and/or the gastric anchor element or balloon segment (8) and/or the duodenal anchor element or balloon segment (6) consist of polyurethane.

14. Bypass device (1) according to any one of the preceding claims, **characterized by** at least one x-ray-opaque marking, which is placed on the transpyloric conducting element (4, 7) and/or in or on the fixing unit (2) proximal and/or distal to the pylorus, to indicate the correct transpyloric location of the transpyloric bypass device (1), in particular the transpyloric conducting element (4, 7) and/or the fixing unit (2).

15. Bypass device (1) according to any one of the preceding claims as part of a system comprising an applicator for the transpyloric placement of the transduodenal bypass device (1) according to any one of the preceding claims in the region of the pylorus of a patient, **characterized by** an applicator element in the form of an endoscope or catheter, having an elongated shaft, on the external surface of which the bypass device (1) can be placed and/or plugged such that the applicator shaft entirely or partially penetrates the central conducting lumen of the bypass device (1).

16. Bypass device (1) according to claim 15, **characterized in that** the bypass device
a) is held reversibly, i.e., detachably, on the applicator shaft, and/or
b) is held by clamping on the applicator shaft, wherein an annular, extracorporeally fillable, gap-bridging balloon is provided for the clamping fixation of the bypass device (1) on the outer side of the applicator shaft, which balloon is located when the bypass device (1) is placed or plugged on, in the annular gap between the applicator shaft, on the one hand, and the transpyloric conducting element (4, 7), on the other hand, in particular radially inside the fixing unit (2) and/or radially inside the front end of the transduodenal conducting tube (7).

17. Bypass device (1) according to any one of claims 15 or 16, **characterized in that** the applicator comprises:
a) a valve for deaerating the balloon clamping the bypass device (1) on the applicator shaft, and/or
b) flushing openings, preferably distal to the gap-bridging balloon, through which liquid can be flushed into the duodenum extracorporeally via a flushing line extending along the applicator, to unfold the distal section of the conducting element (4, 7), and/or
c) at least one x-ray-opaque marking in the region of the distal end, so that the location thereof can be checked as the applicator shaft is advanced.

## Revendications

1. Dispositif de dérivation (1) servant à récupérer le bol alimentaire de l'estomac ou du gaster et et à acheminer de manière dérivative le bol alimentaire par le pylore dans ou à travers le duodénum d'un patient, système comprenant un élément de transfert (4,7) transpylorique tubulaire, repliable radialement et se redressant de lui-même, traversant le pylore, dont un tube central de transfert pour le bol alimentaire, ainsi que des éléments de fixation pour l'ancrage de l'élément de transfert transpylorique (4,7) au pylore, constitué d'un élément d'ancrage gastrique, annelé, disposé du côté de l'estomac ou proximalement par rapport au pylore; pour ancrer l'élément de transfert transpylorique (4,7), proximal au pylore, avec un segment de ballonnet gastrique (8), lequel segment délimite par endroit une cavité de structure annulaire pouvant être remplie d'une substance, ainsi qu'un élément d'ancrage duodénal, annulaire, reposant dans le duodénum, disposé du côté de l'intestin ou distalement par rapport au pylore servant à ancrer l'élément de transfert (4,7) transpylorique distal au pylore, doté d'un segment de ballonnet duodénal (6), qui délimite par endroit une cavité de structure annulaire pouvant être remplie d'une substance, et **caractérisé en ce que** le segment de ballonnet gastrique (8) et/ou le segment de ballonnet duodénal (6) entoure l'élément de transfert transpylorique (4,7) à l'extérieur radial, est non fermé au droit d'une ligne faisant le tour dans le sens toroïdal, et est relié à l'élément de transfert (4,7) de sorte que ce dernier forme une partie de la ceinture de la cavité toroïdale concernée, ce qui l'élément de transfert (4,7) est ainsi constitué ou renforcé, au moins dans la région entourant la cavité gastrique et/ou duodénale, de sorte qu'il présente à cet endroit une stabilité de structure ou une capacité d'auto-redressement plus grand que le segment de ballonnet concerné (6,8) dans les environs immédiats tandis qu'au moins un segment de ballonnet (6,8) (i) présente au moins un secteur du bord libre, s'étendant tout autour, annulairement fermé, adjacente à l'élément de transfert transpylorique (4,7), qui est retourné de sorte qu'il avec sa face intérieure s'orientant vers la cavité concerné s'aplatir au élément de transfert transpylorique (4,7), et (ii) est relié hermétiquement au élément de transfert transpylorique (4,7).

2. Dispositif de dérivation (1), suivant l'une des revendications précédentes, **caractérisé en ce que** les deux bords frontaux libres d'au moins un segment de ballonnet (6,8) sont retournés vers l'intérieur, respectivement dans le segment de ballonnet concerné (6,8), à condition que, de préférence, entre l'un des bords frontaux libres et leur retournement dans le segment de ballonnet concerné (6,8), se situe, à l'intérieur, un deuxième retournement du côté opposé, c'est-à-dire en sortant du segment de ballonnet concerné (6,8).

3. Dispositif de dérivation (1), suivant l'une des revendications précédentes, **caractérisé en ce qu'**un segment de ballonnet (6,8) est préformé de sorte,
a) qu'il présente des longueurs circonférentielles différentes à certains tronçons annulaires déterminées, en particulier qu'il présente à chaque fois une longueur circonférentielle moindre dans la zone des deux bords frontaux libres, que dans une zone intermédiaire de la gaine du ballonnet, qui forme un tronçon se rabattant vers l'extérieur du segment de ballonnet (6,8), et/ou ce de sorte
b) qu'il présente des épaisseurs différentes dans certains tronçons annulaires déterminées, notamment qu'il présente à chaque fois dans la zone des deux bords frontaux libres une épaisseur plus importante que dans l'une des zones intermédiaires de la gaine du ballonnet qui forme un tronçon se rabattent vers l'extérieur du ballonnet (6,8), et/ou ce de sorte
c) que la section transversale présente, en cas de cavité expansée jusqu'à son volume préformé au travers cette même cavité, une plus grande étendue dans le sens axial que dans le sens radial rapporté à l'axe longitudinal de l'élément de transfert (4,7).

4. Dispositif de dérivation (1), suivant l'une des revendications précédentes, **caractérisé en ce qu'**un ou les deux segments de ballonnet (6,8) sont constitué et/ou préformé et fixé au élément de transfert (4,7) de sorte qu'ils ont une tendance à rouler vers le pylore, notamment une tendance à rouler les uns sur les autres.

5. Dispositif de dérivation (1) suivant l'une des revendications précédentes, **caractérisé en ce qu'**à l'intérieur d'au moins une cavité entourée par endroits d'un segment de ballonnet (6,8) est disposé un coussin supplémentaire interne ou un élément de ballonnet qui est ou peut être rempli avec une autre substance de remplissage que celle de la cavité réceptrice elle-même, de préférence à condition que le coussin interne ou l'élément de ballonnet est fixé à l'élément de transfert transpylorique (5,7) hermétiquement sur le pourtour, et/ou renferme un volume moindre et/ou est préformé avec un volume moindre que le segment de ballonnet (6,8) situé à l'extérieur et soutenu par la cavité d'accueil.

6. Dispositif de dérivation (1) suivant la revendication 5, **caractérisé en ce que** l'élément de ballonnet interne:
- peut être rempli avec un liquide, et/ou
- est formé toroïdalement, et/ou
- dans la zone d'une ou des deux bords frontaux annulaires entourant sa face intérieure orientée vers son liquide de remplissage affleure l'élément de transfert transpylorique (5,7) en y étant fixé.

7. Dispositif de dérivation (1) suivant l'une des revendications précédentes, **caractérisé en ce que** le segment de ballonnet gastrique (8) et le segment de ballonnet duodénal (6) se joignent pour ne former qu'un seul ballonnet unique préformé en forme d'haltère, avec un effilement à peu près centré et entourant la prise/entrée du sphincter du pylore.

8. Dispositif de dérivation (1) suivant l'une des revendications précédentes, **caractérisé en ce qu'**au moins un tuyau d'alimentation ou un tuyau de remplissage (22) allant au moins vers une cavité toroïdale, de sorte que peuvent être remplis un ou les deux segments de ballonnet (6,8) après le placement du dispositif de dérivation transpylorique (1), notamment de l'élément de transfert transpylorique (4,7) et/ou l'unité de fixage (2), tandis que de préférence les extrémités orale ou proximale (23) du tuyau de remplissage (22) peuvent être introduites dans l'estomac et/ou être détachées à cet endroit, et/ou tandis que de préférence au moins un tuyau d'alimentation vers au moins une cavité toroïdale, notamment un tuyau de remplissage (22), est pourvu d'un clapet anti-retour.

9. Dispositif de dérivation (1) suivant l'une des revendications précédentes, **caractérisé en ce que** l'élément de transfert transpylorique (4,7) relie l'élément d'ancrage gastrique à l'élément d'ancrage duodénal.

10. Dispositif de dérivation (1) suivant l'une des revendications précédentes, **caractérisé en ce que** l'épaisseur de l'élément de transfert transpylorique (4,7) est plus épais que l'épaisseur du segment de ballonnet gastrique (8) et/ou que l'épaisseur du segment de ballonnet duodénal (6), par exemple est au moins deux fois plus épais, de préférence au moins 5 fois aussi épais, notamment au moins 10 fois aussi épais.

11. Dispositif de dérivation (1) suivant l'une des revendications précédentes, **caractérisé en ce que** l'élément de transfert transpylorique (4,7)
a) est préformé en forme de tuyau de sorte qu'il ne s'effondre pas lors du remplissage d'un ou des deux segments de ballonnet sous l'influence de la pression de remplissage qui y règne, et/ou
b) est ondulé ou plissé, de sorte qu'il ait des propriétés d'auto-redressement, mais de façon à fonctionner comme un soufflet qu'on puisse comprimer ou replier et/ou
c) est raidi dans le secteur d'un ou des deux segments de ballonnet via une douille/manchon ou un ressort en forme d'anneau ou de spirale, de sorte que lors du remplissage un ou les deux éléments de ballonnet ne s'effondrent pas sous la pression de remplissage.

12. Dispositif de dérivation (1) suivant l'une des exigences précédentes, **caractérisé en ce que** l'élément de transfert transpylorique (4,7) qui présente un agencement coaxial à double couche de films tubulaires, tandis que principalement les deux couches du film tubulaire de l'élément de transfert transpylorique (4,7) sont reliées au moins localement l'une à l'autre, principalement via des raccords ponctuels, linéaires ou à fleur, notamment dans le secteur qui traverse le pylore, et/ou principalement là où les couches du film tubulaire de l'élément de transfert transpylorique (4,7) en association avec un ou les deux segments de ballonnet sont formées d'un seul film tubulaire conjoint.

13. Dispositif de dérivation (1) suivant l'une des revendications précédentes, **caractérisé en ce que** l'élément de transfert transpylorique (4,7) et/ou l'élément d'ancrage gastrique ou le segment de ballonnet (8) et/ou l'élément d'ancrage duodénal ou le segment de ballonnet (6) est composé de polyuréthane.

14. Dispositif de dérivation (1) suivant l'une des revendications précédentes, **caractérisé en ce qu'**au moins un marquage radio-opaque, placé sur l'élément de transfert transpylorique (4,7) et/ou sur ou à une unité de fixation (2) proximalement /ou distalement par rapport au pylore pour indiquer l'emplacement correct transpylorique du dispositif de dérivation transpylorique (1), notamment de l'élément de transfert transpylorique (4,7) et/ou de l'unité de fixation (2).

15. Dispositif de dérivation (1) suivant l'une des revendications précédentes, comme composante d'un système avec un applicateur pour un placement transpylorique du dispositif de dérivation transduodénal (1), conformément à l'une des exigences précédentes dans la zone du pylore d'un patient ; **caractérisé en ce qu'**un élément applicateur en forme d'endoscope ou de cathéter, avec une tige allongée sur la surface extérieure de laquelle le dispositif de dérivation (1) peut être posé et/ou enfiché de sorte que la tige de l'applicateur traverse entièrement ou partiellement l'élément de transfert central du dispositif de dérivation (1).

16. Dispositif de dérivation (1) suivant la revendication 15, **caractérisé en ce que** le dispositif de dérivation est maintenu :
a) réversiblement sur la tige d'applicateur, c.-à-d amovible et/ou
b) par serrage sur la tige d'applicateur, tandis que l'on prévoit un ballonnet annulaire extracorporel pouvant être rempli et enjambant une fente pour la fixation serrante du dispositif de dérivation (1) sur la face extérieure de la tige de l'applicateur; ce ballonnet annulaire extracorporel pouvant être rempli et enjambant une fente se situe dans la fente annulaire entre la tige de l'applicateur d'un côté et l'élément de transfert transpylorique de l'autre côté, tout en tenant compte que le dispositif de dérivation (1) doit être posé ou enfiché, notamment radialement au sein de l'unité de fixation (2) et/ou radialement au sein de l'extrémité avant du tuyau passant transduodénal (7)

17. Dispositif de dérivation (1) suivant les revendications 15 ou 16, **caractérisé en ce que** l'applicateur présente :
a) une valve pour la purge du ballon serré/raccordé sur/à la tige de l'applicateur du dispositif de dérivation (1), et/ou
b) des orifices de purge, principalement distaux des ballonnets enjambant une fente via lesquels il est possible d'injecter du liquide extracorporel dans le duodénum en passant par une conduite de rinçage au droit de l'applicateur pour déplier le segment distal de l'élément de transfert (4,7), et/ou
c) au moins un marquage radio-opaque dans le secteur de l'extrémité distale, de sorte qu'en cas d'avancée de la tige de l'applicateur, son bon emplacement peut être vérifié.
